# EUROPEAN PATENT APPLICATION

(11) **EP 4 834 894 A1**
(43) Date of publication of application: **04.11.2026**
(21) Application number: 25305622.0
(22) Date of filing: 30.04.2025
(51) Int. Cl.: C07D 401/04

(54) **USE OF A MECHANICAL FORCE IN THE IMPLEMENTATION OF A PROCESS OF PREPARATION OF IMMUNOMODULATORY DRUGS**

(71) Applicant: Université de Montpellier, 34090 Montpellier (FR); Radboud University, 6525 AJ Nijmegen (NL); Centre National de la Recherche Scientifique, 75016 Paris (FR); Ecole Nationale Supérieure de Chimie de Montpellier, 34296 Montpellier (FR)
(72) Inventor: COLACINO, Evelina, 34090 Montpellier (FR); RUTJES, Floris, 6605 DD WIJCHEN (NL); CHATZIGIANNIS, Christos, 34090 Montpellier (FR); SOLORZANO-RODRIGUEZ, Ruben, 34170 Castelnau-le-Lez (FR); BORDIER, Corentin, 72120 Saint-Calais (FR); ORT, Florian, 6711 NZ EDE (NL); BLANCO-ANIA, Daniel, 3541 DC UTRECHT (NL)
(74) Representative: Cabinet Grosset-Fournier & Demachy

(57) **Abstract**

The invention relates to the use of a mechanical force to generate a mechanochemical reaction in the implementation of a process of preparation of immunomodulatory drugs or a pharmaceutically acceptable salt thereof, and their process of preparation.

## Description

The present invention refers to the use of a mechanical force to generate a mechanochemical reaction in the implementation of a process of preparation of immunomodulatory drugs, and their process of preparation.

### Background of the Invention

Over the past century, intensified human activities involving chemistry have led to significant environmental issues, including ozone layer depletion, global warming, air pollution, and overexploitation of natural resources. In response, several various measures have been implemented over the last 50 years to mitigate the negative environmental impacts of chemical production.

Acknowledging the necessity to lessen the environmental damage caused by the chemical industry has spurred the growth of mechanochemistry.

This approach seeks to safeguard future generations by advancing more sustainable chemical processes. Mechanochemistry allows reactions to occur without solvents or with minimal liquid media, in a process named Liquid Assisted Grinding (LAG), optionally with a solid adjuvant, making it a viable alternative for green chemical synthesis. Its advantages include the absence of bulk solvents, increased kinetics, precise stoichiometric control (using reagents in exact amounts rather than excess), and more selective reactions, which simplify work-up procedures, thus reducing the amount of total waste generated ('Fantozzi, N.; Volle, J-N., Porcheddu, A., Virieux D., Garcia, F., Colacino, E. "Green metrics in mechanochemistry" Chem. Soc. Rev. 2023, 52, 6680 - 6714. DOI: 10.1039/D2CS00997H' and 'Galant, O.; Cerfeda, G.; McCalmont, A.S.; James, S.L.; Porcheddu, A.; Delogu, F.; Crawford, D.E.; Colacino, E.; Spatari, S. "Mechanochemistry can reduce life cycle environmental impacts of manufacturing active pharmaceutical ingredients ACS Sustainable Chem. Eng. 2022, 10, 1430-1439 https://doi.org/10.1021/acssuschemeng.1c06434'). These features highlight the superiority of mechanochemical processes compared to traditional solution-based chemistry.

Thalidomide and pomalidomide are immunomodulatory drugs (IMiDs) that are integral to the treatment of multiple myeloma. Both drugs enhance the immune response by stimulating natural killer cells and modulating cytokine production, which includes increasing interleukin-2 (IL-2) and interferon-gamma levels. This immunomodulatory effect is crucial in combating cancer cells and preventing their proliferation. In the field of cancer treatment, these both drugs bind to cereblon (CRBN), a substrate receptor of the CRL4 E3 ligase complex, leading to the degradation of specific proteins that are essential for myeloma cell survival. The synergistic effects of these IMiDs with other therapies have significantly improved outcomes for multiple myeloma patients, highlighting their importance in modem cancer treatment regimens.

Pomalidomide, a more potent derivative of thalidomide, exhibits also anti-angiogenic effects and is particularly effective in treating relapsed and refractory multiple myeloma. Pomalidomide, approved for use in patients who have received prior therapies, offers a viable option for those whose disease has progressed despite previous other treatments.

Thalidomide, initially used as a sedative, was later recognized for its anti-angiogenic properties, which inhibit the growth of new blood vessels that support tumor growth. In clinical settings, thalidomide is used with dexamethasone or other chemotherapeutic agents to enhance its therapeutic efficacy.

One of the aims of the invention is to provide a new use of a mechanical force to conduct a mechanochemical total synthesis to provide pomalidomide, or pomalidomide derivatives, or a pharmaceutically acceptable salt thereof.

One of the aims of the invention is to provide a new use of a mechanical force to conduct a mechanochemical total synthesis to provide thalidomide, or thalidomide derivatives, or a pharmaceutically acceptable salt thereof.

Another aim of the invention is to provide a mechanochemical process of preparation of pomalidomide, or pomalidomide derivatives, or a pharmaceutically acceptable salt thereof.

Another aim of the invention is to provide a mechanochemical process of preparation of thalidomide, or thalidomide derivatives, or a pharmaceutically acceptable salt thereof.

Additional aim of the invention is the reduction of the amount of starting and intermediate materials in comparison with the manufacturing method in solution.

Another additional aim of the invention is the reduction of the amount of the energy for the heating to carry out the chemical process, and for the cooling which results from it in comparison with the manufacturing method in solution.

Another additional aim of the invention is the short reaction time and the work-up that is easy to implement (no chromatography needed), because of the chemical transformation that is total, in comparison with the manufacturing method in solution.

### Brief summary of the invention

The Applicant has found that a mechanical force to generate a mechanochemical reaction in the implementation of a process of preparation of a compound of formula (I) can be used. Inventors were able to carry out a mechanochemical total synthesis of a compound of formula (I) in 1 or 2 steps, without the use of metal catalysts (with precious metal(s) or not), by implementing mechanochemical processes for its preparation in 1or 2 steps.

### Detailed description of the invention

The invention relates to the use of a mechanical force to generate a mechanochemical reaction in the implementation of a process of preparation in a reactor of a compound of formula (I): 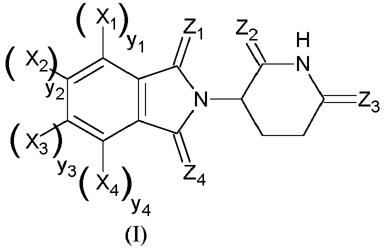 wherein:
- Z₁, Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₅)acyl group, a 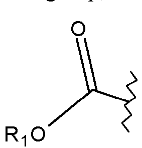 group, or a 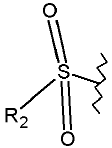 group
   wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
or a pharmaceutically acceptable salt thereof, comprising n steps, n being 1 or 2, from 3-aminopiperidine-2,6-dione hydrochloride or L-glutamine, wherein at least one step is mechanochemical.

As used herein the expression "mechanochemical force" refers to mechanochemical activation or mechanochemistry, that is a technology and a research field in which solid chemical reactants, i.e in the form of granules or powders (pulverulent) or in the pasty state, are milled or ground together or ground together under mechanical activation and the chemical reactions are induced by the direct absorption of mechanical energy, (McNaught, A.D.; Wilkinson, A. IUPAC Compendium of Chemical Terminology ("The Golden Book"); 2nd edition; Blackwell Scientific publications, Oxford, 1997) without solvents (neat grinding) or with minimum/small amounts of solvent in a process named liquid-assisted grinding (LAG) in comparison with solution-based methods. The mechanochemical reaction occurs because of the activation by the mechanical force.

As used herein, the term "liquid-assisted grinding (LAG)" refers to minimum or limited amounts of a liquid, the role of which is to increase ease of mixing and/or to participate to the reaction occurring in a mechanochemical system. Under conventionally defined LAG conditions; the liquid additive has also been described as functioning essentially as a catalyst or lubrificant.

LAG uses a small amount of a liquid to accelerate reactions, as well as to enable transformations that do not take place by neat grinding. The empirical definition of LAG is based on the way mechanochemical reactivity is affected by the ratio of the liquid additive to the weight of chemical reactants (η, eta).

A value of η = 0 µL/mg corresponds to neat grinding, η > 2 µL/mg corresponds to a typical solution reaction, while LAG lies in the range of ≈0-1 µL/mg. In that range, reactivity appears independent of reactant solubility, distinguishing LAG conditions from slurry reactions (1 µL/mg < η < 2 µL/mg) in which low solubility does hinder reactivity. (Friščić et al., *ACS Cent. Sci.* **2017,** 3(1), 13-19)

As used herein, the terms "minimum or limited amounts of a liquid" or "minimum /small amount of solvent" refers to an eta value (*η*) up to 1, compared to the same reaction without mechanochemical conditions".

The compounds involved in the Invention are the ones of formula (I), their enantiomers, in particular under pure form, or a mixture of said enantiomers, in particular a racemic mixture.

The formula (I) encompasses compounds of formula (III): 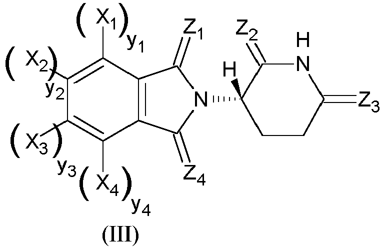 wherein Z₁, Z₂, Z₃, Z₄, X₁, X₂, X₃, X₄, y₁, y₂, y₃ and y₄ have the meaning defined above and compounds of formula (IV): 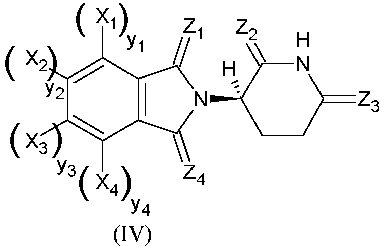 wherein Z₁, Z₂, Z₃, Z₄, X₁, X₂, X₃, X₄, y₁, y₂, y₃ and y₄ have the meaning defined above.

The expression "(C₁-C₅)alkyl" group means an alkyl group having from 1 to 5 carbon atoms.

The expression "(C₁-C₅)alkoxy" group means an alkyl group from 1 to 5 carbon atoms, which is singularly bonded to oxygen.

The expression "(H or C₁-C₅)acyl group" means a C₁ - C₅ 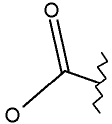 group.

The expression" halogen atom" means an atom chosen among: fluorine, chlorine, bromine or iodide.

The expression "pharmaceutically acceptable salt" means all pharmaceutically or physiologically acceptable salt forms of the compounds of compound of formula (**I**) which may be formed, by protonation of a nitrogen, with an inorganic or organic acid. Examples acid addition salts comprise, mineral acid salts such as hydrochloride, hydrobromide, hydroiodide, sulfate salts (such as, e.g., sulfate or hydrogensulfate salts), phosphate salts (such as hexafluorophosphate, hydrogenphosphate, or dihydrogenphosphate salts), carbonate salts, hydrogenocarbonate salts, borate salts, organic acid salts such as oxalate, fumarate, tartrate, malate, citrate, succinate, adipate, gluconate, glycolate, nicotinate, benzoate, salicylate, ascorbate salts; sulfonate salts such as methanesulfonate (mesylate), ethanesulfonate (esylate), 2-hydroxyethanesulfonate (isethionate), p-toluenesulfonate (tosylate), or camphorsulfonate salts; glycerophosphate salts; and acidic amino acid salts such as aspartate or glutamate salts. Preferred pharmaceutically/physiologically acceptable salts of compound of formula (I) include hydrochloric acid, hydrobromic acid, methane sulfonic acid, ethane sulfonic acid, benzene sulfonic acid and p-toluenesulfonic acid.

The starting product in the preparation of a compound of formula (I) is:
- 3-aminopiperidine-2,6-dione hydrochloride (CAS number: 24666-56-6) is a salt of a cyclic dione compound containing two nitrogens; it consists of a six-membered ring, in presence with a hydrochloride salt,
   or
- L-glutamine, (acide 2-amino-5-carbamoylpentanoique, CAS number: 56-85-9) is a natural α-amino acid.

The term "wherein at least one step is mechanochemical" refers to the fact that in the process of preparation of compound of formula (I), the minimum number of steps that is performed thanks to mechanical activation is 1.

According to another embodiment, the invention consists in the use of a mechanical force to generate a mechanochemical reaction in the implementation of a process of preparation, of a compound of formula (I) or a pharmaceutically acceptable salt thereof, said process comprising 2 steps, using 3-aminopiperidine-2,6-dione as starting material, wherein at least one step is mechanochemical.

According to another embodiment, the invention consists in the use of a mechanical force to generate a mechanochemical reaction in the implementation of a process of preparation, of a compound of formula (I) or a pharmaceutically acceptable salt thereof, said process comprising 1 step, using L-glutamine as starting material, wherein at least one step is mechanochemical.

This embodiment is advantageous because L-glutamine is a very widely used and cheap chemical compound.

According to a particular embodiment of the use of the invention, the process of preparation comprises a first step which is a cycloimidation step (which is mechanochemical) and a second step of mechanochemical reduction.

According to a particular embodiment, the present invention concerns the preparation of compound of formula (I) chosen among (Ia) or (Ib):

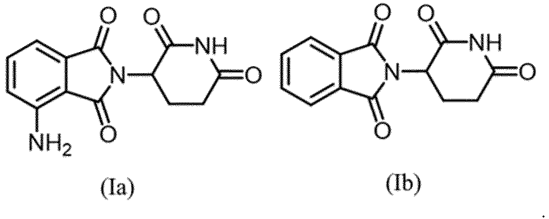

When the compound of formula (I) is the compound of formula (Ia), then y₁ = y₂ = y₃ = 0, y₄ = 1, Z₁, Z₂, Z₃ and Z₄ are oxygen atoms and X₄ is an amino group.

The compound of formula (Ia) corresponds to pomalidomide (CAS number: 19171-19-8).

When the compound of formula (I) is the compound of formula (Ib), then y₁ = y₂ = y₃ = y₄ = 0, Z₁, Z₂, Z₃ and Z₄ are oxygen atoms.

The compound of formula (Ib) corresponds to thalidomide (CAS number: 50-35-1).

According to a particular embodiment of the invention, said process is carried out in batch, in continuous or in semi-continuous.

The expression "batch" means a type of process carried out in a closed system, where the totality chemical reactants are added at the beginning, and the process proceeds without further input or removal of materials until the end of the process.

The expression "continuous" means is a type of process carried out in a continuously flowing system, where reactants are introduced and products are removed simultaneously.

The expression "semi-continuous" means a combination of both batch and continuous. The totality of chemical reactants is processed in batches but then linked together in a continuous sequence. This means no breaks during the process, and total chemical reactants are constantly fed into the system.

According to a particular embodiment of the invention, the mechanical forces are generated by a method chosen among:
o milling, using a milling device, in particular vibrating ball-milling (VBM) or planetary ball-milling (PBM),
o resonant acoustic mixing (RAM),
o screw extrusion (single, twin or multi).

Concerning the milling, examples of the reaction vessel and the balls as an agitation medium used in the milling devices include ones formed of such materials as stainless steel, agate, teflon (PTFE), alumina, tungsten carbide, chrome steel, zirconium oxide, silicon nitride and any material adapted to mechanochemical forces. Among these materials, zirconium oxide, is preferred. The size of the vessel used in the planetary ball mill is not particularly limited and may be approximately 1 cm³ to 1000 L. The size of the balls is also not particularly limited and may be approximately from 2 to 50 mm in diameter. Particularly preferred specific examples of the vibrating and/or planetary ball mill include stainless steel and zirconia oxide as reaction vessel material.

Resonant acoustic mixing (RAM) involves applying acoustic energy at a frequency of 60 Hz while performing a vibrating oscillating movement reciprocating up and down motions on the contents of the container. This movement may be applied with a relatively high (up to 100 g) acceleration and a relatively low amplitude. The frequency of the vibration is typically around 60 Hz. The container can be cylindrical shaped with a circumferential side wall and disc-shaped top and bottom walls. The force is applied along the longitudinal axis of the container.

In resonant acoustic mixing, acoustic energy is delivered to the components to be mixed. An oscillating mechanical driver creates motion in a mechanical system comprising engineered plates, eccentric weights and springs. This energy is then acoustically transferred to the material to be mixed. The underlying technology principle is that the system operates at resonance. In this mode, there is a nearly complete exchange of energy between the mass elements and the elements in the mechanical system.

The resonant acoustic mixing provides a highly efficient way of transferring mechanical energy directly into the starting materials. The resonant frequency can be from about 15 Hertz to about 2000 Hertz, or from about 20 Hertz to about 1800 Hertz, or from about 20 Hertz to about 1700 Hertz. The g force applied by the acoustic mixer to the reactant composition can be from about 2 g force to about 100 g force.

Resonant acoustic mixers are available from Resodyn^{™} Acoustic Mixers and can be chosen from LabRAM I and LabRAM II, RAM5, RAM55, OmniRAM and like.

Screw extrusion may be performed at various speeds (S_{S}), screw temperatures (S_{T}) and residence times (T_{R}), as described herein. A single pass through the extruder may be sufficient to form the product of a mechanochemical reaction. Alternatively, when step a) is conducted in a twin-screw extruder, step a) may comprise collecting the product emerging from the twin screw extruder and subjecting it to one or more additional passes through the simple or twin-screw extruder.

Multi-screw extruders for use herein are not particularly limited. Any multi-screw extruder comprising a reaction chamber commonly known in the art may be used in the context of the present disclosure. Suitable multi-screw extruders for use herein will be easily identified by those skilled in the art, in the light of the present description.

In a particular aspect of the present application, the screw extruder for use herein is selected from the group of Single Screw Extruders (Helibar Transfer Mix, Co-Kneader), Twin Screw Extruders: 1. Intermeshing Screws: Co-rotating Parallel Screws, Counter-rotating Parallel Screws), 2: Non-intermeshing Screws (Co-rotating Parallel Screws, Co-rotating Conical Screws, Counter-rotating Screws), 3: Multiple Screw Extruders (Rotating Center Shaft: Planetary Roller Extruder), Static Center Shaft (Ring Extruder, Multiple Screw Extruder).

The main extruder parameters to consider are:
- feed rate of chemical reactants forming the starting material mixture: from 0.1 g/min to 10 g/min for an extruder that is adapted to a laboratory (as a matter of example and in a non-limiting manner, with 9 mm, (length/diameter ratio) 25:1 TSE; from 0.4 to 1.5 g/min) and for larger extruders in the industry, from 1 kg/min to 50 kg/min)
- Number of the liquid inlet ports and their position (as a matter of example and in a non-limiting manner with ZE9 Three-Tec extruder (LD 25: 1) of Three Tec company, 2 liquid ports are available: one close to the beginning of the barrel and one close to the end) used for the addition of liquid additive (the flow rate of liquid additive depends on the eta value and the feed rate of solid chemical reactants)
- Presence or not of a die and the type of die (The dies are defined and manufactured according to process requirements. Die diameters are possible from 0.1 mm. The empty volume in the die is negligible, as the screws extend into the die until just before the opening. Due to the simple design and low mass, separate die heating can be dispensed with in most cases. If heating of the die is nevertheless necessary, the same heating elements as for the barrel can be used for this purpose. The following die geometries are possible: Single and double dies, Sieve dies, Film dies (fixed or adjustable), Hose dies, Cooling dies for meat substitutes).
- Screw speed (from 20 to 1000 RPM) (as a matter of example and in a non-limiting manner Hybrid ZE9/ZE12 Three-Tec extruder of Three Tec company can rotate from 20 to 200 RPM).
- Barrel temperature (as a matter of example and in a non-limiting manner, the hybrid ZE 9/12 Three-Tec TSE extruder of Three Tec company is able to heat from 25°C to 400°C, more preferably from 25°C to 230 °C.
- Screw profile: Modular allow infinite configuration of screw profiles by replacing some screw elements by others. Screw elements can be: conveying element (reverse or not), mixing elements (reverse or not) and kneading elements.

According to a particular embodiment of the invention, one step is mechanochemical.

According to a particular embodiment of the use of the invention, two steps are mechanochemical.

According to a particular embodiment of the use of the invention, said process is carried out in flow and wherein at least one step is mechanochemical.

According to a particular embodiment of the use of the invention, said process is carried out in flow and wherein at least two steps are mechanochemical.

According to a particular embodiment of the invention, said process of preparation can provide at least 4.10³ kg.m⁻³ of said reactor.day⁻¹, in particular from 4.10³ kg.m⁻³ of said reactor.day⁻¹ to 68 000 kg.m⁻³ of said reactor.day⁻¹, of said compound of formula (I) or of said pharmaceutically acceptable salt thereof.

By range of "from 3.10³ kg.m⁻³ of said reactor.day⁻¹ to 68 000 kg.m⁻³" it means: from 3 000 to 4 000, from 4 000 to 8 000, from 8 000 to 12 000, from 12 000 to 16 000, from 16 000 to 20 000, from 20 000 to 24 000, from 24 000 to 28 000, from 28 000 to 32 000, from 32 000 to 36 000, from 36 00 to 40 000, from 40 000 to 44 000, from 44 000 to 48 000, from 48 000 to 52 000, from 52 000 to 56 000, from 56 000 to 60 000, from 60 000 to 64 000 and from 64 000 to 68 000 kg.m⁻³ of said reactor.day⁻¹.

According to a preferred embodiment of the invention, the process comprises a cycloimidation step of 3-aminopiperidine-2,6-dione hydrochloride into 2-(2,6-dioxopiperidin-3-yl)-4-nitroisoindoline-1,3-dione with the use of 3-nitrophthalic acid, CDI (1,1'-carbonyldiimidazole), a base generated in situ by CDI and an additive.
and/or
comprises a step of mechanochemical reduction of the above mentioned 2-(2,6-dioxopiperidin-3-yl)-4-nitroisoindoline-1,3-dione into a compound of formula (Ia) 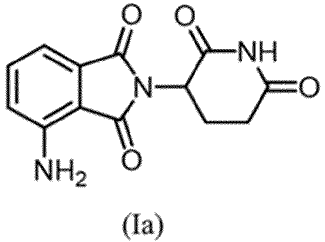
with at least one additive and sodium dithionite.

According to a preferred embodiment of the invention, the process comprises a cycloimidation step of 3-aminopiperidine-2,6-dione hydrochloride with phthalic anhydride, CDI (1,1'-carbonyldiimidazole) and a base generated in situ by CDI,
into a compound of formula (Ib)

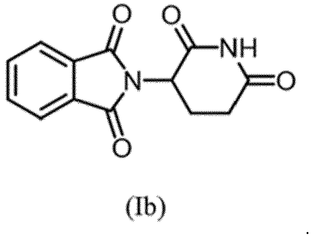

According to a preferred embodiment of the invention, the process comprises a cycloimidation step of L-glutamine with phthalic anhydride, CDI (1,1'-carbonyldiimidazole) and a base generated in situ by CDI,
into a compound of formula (Ib)

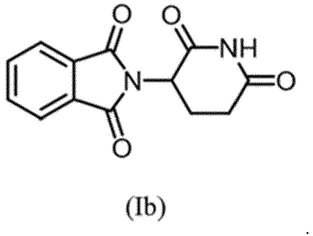

The cycloimidation step in this embodiment consists in the formation of two amide functions with a single nitro atom and a cyclization, represented by one of the following schemes: 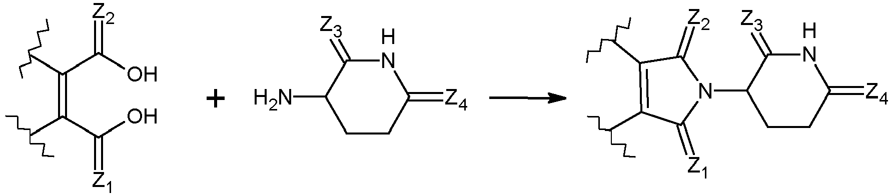 or 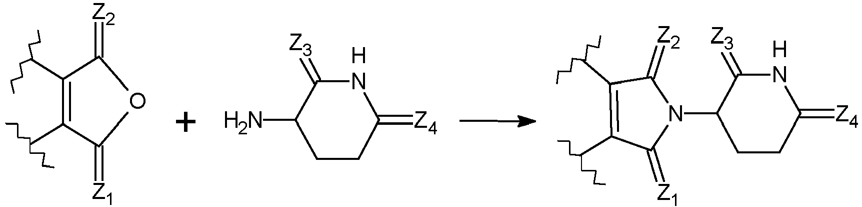 or 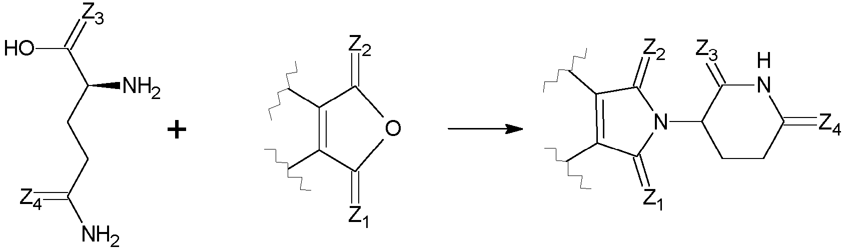 wherein Z₁, Z₂, Z₃ and Z₄ have the meaning defined above.

An additional advantage of the invention is the cycloimidation reaction which in solution occurs in about 17h, when according to the Invention the mechanochemical reaction lasts about 2h to 3h using milling and RAM.

Concerning the step of mechanochemical reduction, it consists in the reduction of at least one nitro group into an amino group, represented in a non-limiting manner by the following scheme: 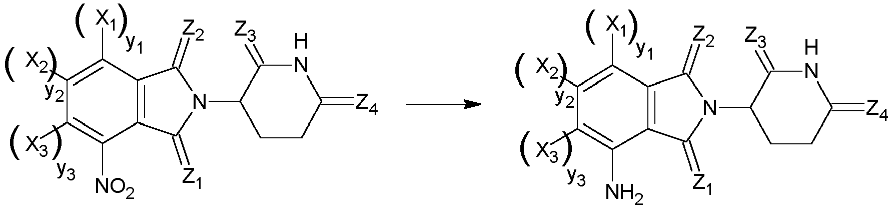 wherein:
- Z₁, Z₂, Z₃ and Z₄ having the meaning defined above,
- X₁, X₂ and X₃, identical or different, are chosen among: (C₁-C₅)alkyl group, (C₁-C₅)alkoxy group, halogen atom, trifluoromethyl group, hydroxy group, (H or C₁-C₁₀)acyl group, a 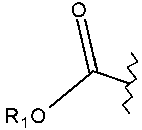 group, or a 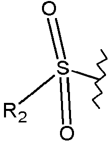 group
   wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
y₁, y₂, y₃ and y₈, identical or different, are equal to 0 or 1, and provided that y₁ + y₂ + y₃ is 1.

Another object of the present invention is the mechanochemical process of preparation of compound of formula (I).

In a particular embodiment, the invention is a batch, continuous or semi-continuous mechanochemical process of preparation of a compound of formula (I), 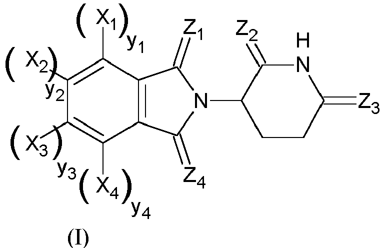 wherein:
- Z₁, Z₂, Z₃ and Z₄, identical or different, are chosen among: oxygen or sulfur atom,
- X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₅)acyl group, a 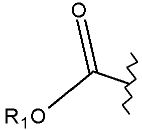 group, or a 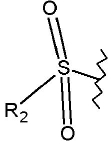 group
   wherein R₁ is chosen among: hydrogen atom, (C₁-C₅)alkyl group, or -NR₁ₐR_{1b} group,
   R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
a) a cycloimidation step using 3-aminopiperidine-2,6-dione hydrochloride or L-glutamine as starting material, in the presence of
   a carboxylic function bearer chosen among 3-nitrophthalic acid, 3-nitrophthalic anhydride, phthalic acid or phthalic anhydride, and
      optionally a coupling agent,
      optionally a base, possibly generated *in situ* by said coupling agent, in particular chosen among tripotassium phosphate and potassium carbonate,
      optionally at least one additive, in particular said at least one additive being under the form of a liquid or of a solid,
   said cycloimidation step being carried out under neat grinding, or in the presence of a solid adjuvant, or in liquid-assisted grinding (LAG) conditions, or in LAG conditions with a solid adjuvant,
   to obtain a crude product containing a compound of formula (II): 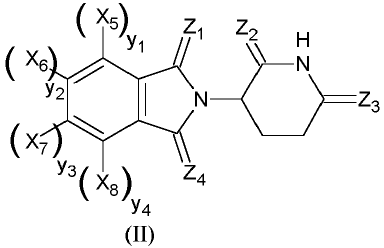 wherein:
      - Z₁, Z₂, Z₃ and Z₄, identical or different, are chosen among : oxygen or sulfur atom,
      - X₅, X₆, X₇ and X₈, identical or different, are chosen among: (C₁-C₅)alkyl group, (C₁-C₅)alkoxy group, nitro group, halogen atom, trifluoromethyl group, hydroxy group, (H or C₁-C₅)acyl group, a 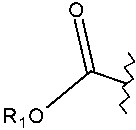 group, or a 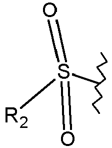 group
         wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
         and R₂ is chosen among: hydroxy group, (C₁-C₅)alkoxy group, -NR₂ₐR_{2b},
         R₂ₐ and R_{2b}, identical or different, are chosen among: hydrogen atom or (C₁-C₅)alkyl group
      - y₁, y₂, y₃ and y₄, identical or different, are equal to 0 or 1, and provided that y₁ + y₂ + y₃ + y₄ is strictly inferior to 3,
      said process comprising optionally,
b) a step of mechanochemical reduction of the above-mentioned compound of formula (II), with at least one additive, using mechanochemical forces, in liquid-assisted grinding (LAG) conditions with sodium dithionite, to obtain a crude product containing said compound of formula (I).

The expression "carboxylic function bearer" refers to a compound bearing at least one carboxylic acid function, or which is an organic acid anhydride.

Mechanochemistry reactions can require the addition of an adjuvant to improve the rheology for efficient reactivity. It has been shown that different textures of reaction mixtures can lead to different reaction kinetics, the reaction mixture can in some cases become sticky and resembles a paste, this prevents efficient mass and energy transfer. To take this into account, it is common to use a solid adjuvant such as silica, alumina, talc, celite or inorganic salts (e.g., NaCl or NaBr) and other chemically inert salts as a milling agent/auxiliary or adsorbent.

Solid additives have the function to improve the rheology of the reaction during the mechanochemical process.

Liquid additive can also be selected in the group of any solvent, polar or apolar (hexane, ethyl acetate, acetone, acetonitrile, dimethyl sulfoxide, dimethyl isosorbide, C₁-C₁₀ alcohol, isopropanol), bio-sourced or not, low melting polymers, deep eutectic solvents (DES), including ionic liquids of any type, such as and in a non-limiting manner, DES choline chloride/glycerol (1 : 2, mol/mol), also known as glyceline or choline chloride/ethylene glycol (1 : 2, mol/mol).

The expression "neat grinding" corresponds to the mechanical force that takes place in absence of solid or liquid additive.

The expression "or in the presence of a solid additive" refers to the mechanical force that takes place in the presence of a solid additive (e.g., adjuvant), with or without a liquid additive.

The expression "a crude product containing" means that during one of the step a) or b), a crude product containing various traces of unwanted reagents is obtained, and this crude product must go through a purification to give the desired product.

The expression "LAG conditions with a solid additive" refers to the mechanical force that takes place in the presence of a liquid additive that improves the mixing of the reaction and may/may not participate in the reaction mechanism in a catalytic way and where the rheological properties of the mixture are improved by the presence of a solid additive.

The expression "a base, possibly generated *in situ* by said coupling agent" designates the case when a coupling agent is present, and that coupling agent is able to generate a base during the step a). As a matter of example and in a non-limiting manner, when CDI is the coupling agent, then, imidazole is generated *in situ* and plays the role of a base.

In the case of milling, using for example NaCl as adjuvant avoids the presence of impurities and improves the rheology of the reaction mixture. In the case of RAM activated reactions, the solid additive is not necessary and only liquid additives in the process is needed. In both cases (milling and RAM), the work-up procedures are simplified, because the product can be easily recovered avoiding purifications steps, otherwise needed when impurities are formed. This has also the advantage to improve the environmental footprint of the entire process, also reducing the amount of waste generated.

According to a particular embodiment of the batch, continuous or semi-continuous mechanochemical process of the invention, the step a) is carried out with at least one additive, said additive being a solid additive or a liquid additive.

According to a particular embodiment of the batch, continuous or semi-continuous mechanochemical process of the invention, the step a) is carried out with at least one additive, and:
- when said at least one additive is solid under mechanical forces, the step a) is carried out in the presence of a solid adjuvant,
   or
- when said at least one additive is liquid under mechanical forces, the step a) is carried out in liquid-assisted grinding (LAG) conditions,
   or
- when said at least one additive comprises a solid adjuvant and a liquid additive under mechanical forces, the step a) is carried out in LAG conditions with a solid adjuvant.

In this embodiment, there is an additive (for LAG) with or without an adjuvant for rheology purposes. This embodiment corresponds to all the transformations, in dependently on the device used for the reactions.

The terms "when said at least one additive is solid under mechanical forces," refers to the case where the at least solid additive is under the form of a solid during the process.

The terms "when said at least one additive is liquid under mechanical forces" refers to the case where the at least solid additive is under the form of a liquid during the process. This case also comprises the case where the additive is solid before the beginning of the process, and becomes liquid under mechanochemical forces. As a matter of example and in a non-limiting manner, the liquid additive is polyethylene glycol 2000 (PEG-2000), which is solid at 25°C.

The terms "when said at least one additive comprises a solid additive and a liquid additive under mechanical forces" refers to the case where at least the solid adjuvant is under the form of a solid during the process and where at least the liquid additive is under the form of a liquid during the process. This case also comprises the case where the additive is solid before the beginning of the process and becomes liquid under mechanochemical forces.

According to a particular embodiment in the process of the invention, the step a) and/or the step b) are carried out with a liquid additive in liquid-assisted grinding (LAG) conditions.

According to a particular embodiment of the batch, continuous or semi-continuous mechanochemical process of the invention, the step a) is carried out with 3-nitrophthalic acid, 3-nitrophthalic anhydride, phthalic acid or phthalic anhydride as carboxylic function bearer, and in absence of a coupling agent.

In this embodiment, the absence of coupling agent leads to a more straightforward process that reduces the overall cost of the process and the number of byproducts, leading to an enhancement of purity of the said compound of formula (I).

According to a particular embodiment of the batch, continuous or semi-continuous mechanochemical process of the invention, the ratio of carboxylic function bearer: additive is from 0.01 to 10, in particular 0.10.

By range of "from 0.01 to 10" it means: from 0.01 to 0.10, from 0.10 to 1 and from 1 to 10".

According to a particular embodiment of the batch, continuous or semi-continuous mechanochemical process of the invention, in step a) and/or in step b), the mechanical forces are generated by a method chosen among:
∘ milling, using a milling device, in particular vibrating ball-milling (VBM) or planetary ball-milling (PBM),
∘ resonant acoustic mixing (RAM),
o screw extrusion (single, twin or multi).

The advantages of this embodiment are:
- for step a) and step b) wherein the mechanical forces are generated by milling:
   1) to reduce the reaction times to 2h (as a matter of example, examples 1 and 3 respectively), compared to the manufacturing process in solution, which requires 17 h for pomalidomide.
   2) to avoid the use of (toxic) organic bases (e.g., triethylamine), which are herein replaced by the inorganic (non-toxic) base or being generated *in-situ,* leading to an improved eco footprint of the process;
   3) to avoid the heating at 80°C, the reaction occurring at room temperature, making the process less-energy demanding;
   4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.
- for step a) wherein the mechanical forces are generated by RAM:
   1) to reduce the reaction times to 2h or 3h compared to the manufacturing process in solution, which requires 17 h for pomalidomide
   2) to avoid the use of (toxic) organic bases (e.g., triethylamine), which are herein replaced by the inorganic (non-toxic) bases (*e.g*., K₂CO₃, K₃PO₄) leading to an improved eco footprint of the process;
   3) to avoid the heating at 80°C, the reaction occurring at room temperature, making the process less-energy demanding;
   4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.
- for step b) wherein the mechanical forces are generated by RAM:
   1) to reduce the reaction times to 3h compared to the manufacturing process in solution, which requires 17 h.
   2) to circumvent the use of precious, toxic and costly raw materials such as metal-based catalysts, for which shortage is also an issue (e.g., palladium catalyst), also making the recovery of the final product cumbersome (for the catalyst separation) and generating toxic waste. The reduction by RAM is 'metal-free', make use cheap reagent (e.g. Na₂S₂O₄), generate not-harmful waste, leading to an improved eco-footprint of the process ; in particular, during the process of the Invention, NaHSO₃ is formed as byproduct. NaHSO₃ is also used in food processing as sanitising agent for food containers and fermentation equipment, as preservative to reduce or prevent microbial spoilage, as selective inhibitor of undesirable microorganisms in the fermentation industries, and as an antioxidant and inhibitor of enzyme-catalysed oxidative discoloration;
   3) to avoid the heating at 50-60°C, the reaction occurring at room temperature, making the process less-energy demanding;
   4) to avoid the use of harmful (and high boiling temperature) solvents such as 1,4-dioxane.
- for step a) and step b) wherein the mechanical forces are generated by TSE:
   1) to avoid the use of (toxic) organic bases (e.g., triethylamine), which are herein replaced by the inorganic (non-toxic) base (*e.g*., K₂CO₃) leading to an improved eco footprint of the process;
   2) to avoid the use of any base in the case wherein the extruder generating the mechanical forces is resistant to corrosion
   3) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.
   4) to avoid the use of solvent, including during work-up (for step a) only)
   5) to avoid the use of a coupling agent
   6) to use a stoichiometric amount of reagent (no excess required)
   7) to reach excellent green chemistry metrics (atom economy, E-factor, Process Mass Intensity (PMI) for example) compared to the solution-based process

According to a particular embodiment of the process of the invention, when the mechanical forces are generated by RAM, said mechanochemical process is carried out in a vessel wherein a m x *g* force is applied, m being a positive number.

In this embodiment, the vessel is made of a material chosen, as a matter of example and in a non-limiting way among: glass, pyrex^{®}, polypropylene or stainless steel, and has a volume comprised from 2 mL to 2 L.

According to a particular embodiment of the batch, continuous or semi-continuous mechanochemical process of the invention, when the mechanical forces are generated by RAM, said mechanochemical process is carried out in a vessel wherein a m x *g* force is applied, m being a positive number, in particular from about 2 g to 100 g, in particular 90 g.

According to a particular embodiment of the batch, continuous or semi-continuous mechanochemical process of the invention, the g force is from about 2 g to about 100 g.

According to a particular embodiment of the process of the invention, when the mechanical forces are generated by RAM, said mechanochemical process is carried out in a vessel wherein a m x *g* force is applied, m being a positive number.

In this embodiment, the vessel is made of a material chosen, as a matter of example and in a non-limiting way among: glass, pyrex^{®}, polypropylene or stainless steel, and has a volume comprised from 2 mL to 2 L.

According to a particular embodiment, the batch, continuous or semi-continuous mechanochemical process of the invention enables the preparation of a compound of formula (III): 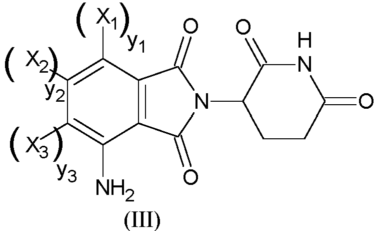 in particular of formula 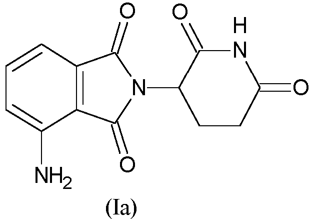 wherein X₁, X₂, X₃, y₁, y₂ and y₃ have the meaning defined above, said process comprising:
a) a cycloimidation step, wherein 3-aminopiperidine-2,6-dione hydrochloride is used as starting material, in the presence of
   a carboxylic function bearer chosen among 3-nitrophthalic acid or 3-nitrophthalic anhydride,
   a coupling agent chosen among carbonyldiimidazole (CDI), *N,N,N,N-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-ethyl-3-(3-dimethylaminopropyl)carbodiimide (EDC),
   and optionally a base, possibly generated *in situ* by said coupling agent,
      optionally at least one additive is used, in particular dimethyl sulfoxide or dimethyl isosorbide, or a mixture of thereof,
   and said mechanical forces are generated by a milling device, and said cycloimidation step is carried out under neat grinding or in LAG conditions,
   to obtain a crude product containing a compound of formula (IV), 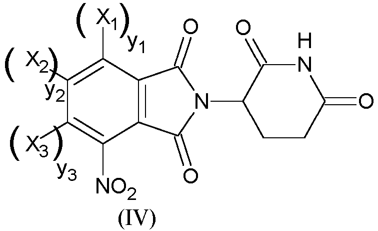
   wherein X₁, X₂, X₃, y₁, y₂ and y₃ have the meaning defined above,

   wherein when the step a) is in LAG conditions, the latter are defined by the parameter η that is greater than 0 µL/mg and less than or equal to 1 µL/mg, more preferably is comprised from about 0.1 µL/mg to 0.3 µL/mg, more preferably 0.2 µL/mg,
   said process comprising
b) a step of mechanochemical reduction of the above-mentioned compound of formula (IV), said mechanical forces being generated by a milling device, to obtain a crude product containing said compound of formula (III), wherein in the step b), the LAG conditions are defined by the parameter η that is greater than 0 µL/mg and less than or equal to 1 µL/mg, more preferably is comprised from about 0.25 µL/mg to 0.75 µL/mg, more preferably 0.5 µL/mg.

According to a particular embodiment of the batch, continuous or semi-continuous mechanochemical process of the invention, when the step a) is in LAG conditions, the additive is liquid and is dimethyl sulfoxide.

According to a particular embodiment of the batch, continuous or semi-continuous mechanochemical process of the invention, the step a) is carried out in the absence of a base.

According to a particular embodiment of the batch, continuous or semi-continuous mechanochemical process of the invention, when the step a) is in LAG, the said at least one additive comprises a liquid additive chosen among: ethyl acetate, γ-valerolactone, dimethyl isosorbide or a mixture of thereof.

According to a particular embodiment of the batch, continuous or semi-continuous mechanochemical process of the invention, the step a) is in the presence of solid additive, in particular polyethylene glycol 2000.

According to a particular embodiment of the batch, continuous or semi-continuous mechanochemical process of the invention, when the step b) is in LAG conditions, the said at least one additive is liquid and is a mixture of water and an alcohol, in particular ethanol.

As a matter of example and in a non-limiting manner and according to a particular embodiment of the batch, continuous or semi-continuous mechanochemical process of the invention, in step a) and/or the step b, the milling speed range value is chosen from 1 to 6000 rpm, or from 1 to 100 Hz.

The milling speed value in rpm can be converted into hertz by applying the following formula: Milling speed (in Hz) = Milling speed (in rpm) / 60

According to a particular embodiment of the batch mechanochemical process of the invention, in step a), the milling device is chosen among VBM or PBM

As a matter of example and in a non-limiting manner and according to a particular embodiment of the batch mechanochemical process of the invention, in step a), the milling device is VBM and the milling speed value is chosen from 5 to 65 Hz, in particular 35 Hz.

As a matter of example and in a non-limiting manner and according to a particular embodiment of the batch mechanochemical process of the invention, in step a), the milling device is PBM and the milling speed value is chosen from 1 to 1100 rpm, in particular 450 rpm.

According to a particular embodiment of the batch mechanochemical process of the invention, in step b), the milling device is chosen among VBM or PBM.

As a matter of example and in a non-limiting manner and according to a particular embodiment of the batch mechanochemical process of the invention, in step b), the milling device is VBM and the milling speed value is chosen from 5 to 65 Hz, in particular 35 Hz.

As a matter of example and in a non-limiting manner and according to a particular embodiment of the batch mechanochemical process of the invention, in step b), the milling device is PBM and the milling speed value is chosen from 1 to 1100 rpm, in particular 450 rpm.

According to a particular embodiment of the batch, continuous or semi-continuous mechanochemical process of the invention, in step a) the cycloimidation is carried out under neat grinding and is performed at a temperature from 50°C to 100°C, in particular 75°C.

According to a particular embodiment of the batch, continuous or semi-continuous mechanochemical process of the invention, the compound of formula (IV) is recovered from the crude product containing it, by a washing with water, a filtration and a removal of said water.

According to a particular embodiment of the batch, continuous or semi-continuous mechanochemical process of the invention, the compound of formula (III) is recovered from the crude product containing it, by a washing with boiling solvent, in particular acetone, a filtration and an evaporation of said acetone.

According to a particular embodiment of the batch, continuous or semi-continuous mechanochemical process of the invention, the compound of formula (III) is recovered from the crude product containing it, by refluxing it with a hydrochloric acid solution, a filtration, a washing with a solution of NaHCO₃ and distilled water, a drying, and an evaporation of said water.

According to a particular embodiment of the batch, continuous or semi-continuous mechanochemical process of the invention, the compound of formula (III) is recovered from the crude product containing it, by an introduction of gaseous hydrochloric acid, and a washing with NaHCO₃ or an introduction of gaseous NH₃.

As a matter of example and in a non-limiting manner and according to a particular embodiment of the batch, continuous or semi-continuous mechanochemical process of the invention, in step b), the milling device is an eccentric mill, said eccentric mill comprises a milling chamber and an oscillation circuit, and the milling speed value is chosen from 10 to 2000 rpm.

According to a particular embodiment of the batch, continuous or semi-continuous mechanochemical process of the invention, the oscillation circuit range is chosen from 6 to 15 mm.

As a matter of example and in a non-limiting manner, the step a) can be carried out with a planetary ball-milling operating at 20-22°C, with 3-nitrophthalic acid as the carboxylic acid bearer, 3-aminopiperidine-2,6-dione hydrochloride as the starting material, CDI as a coupling agent and dimethyl sulfoxide as liquid additive in LAG conditions.

As a matter of example and in a non-limiting manner, the step a) can be carried out with a vibrating ball-milling operating at 20-22°C, with 3-nitrophthalic acid as the carboxylic acid bearer, 3-aminopiperidine-2,6-dione hydrochloride as the starting material, CDI as a coupling agent and dimethyl sulfoxide as liquid additive in LAG conditions.

As a matter of example and in a non-limiting manner, the step b) can be carried out with a planetary ball-milling operating at 20-22°C, with 2-(2,6-dioxopiperidin-3-yl)-4-nitroisoindoline-1,3-dione as compound of Formula (IV), sodium dithionite, water and ethanol as liquid additive in LAG conditions. As a matter of example and in a non-limiting manner, the step b) can be carried out with a vibrating ball-milling operating at 20-22°C, with 2-(2,6-dioxopiperidin-3-yl)-4-nitroisoindoline-1,3-dione as compound of Formula (IV), sodium dithionite, water and ethanol as liquid additive in LAG conditions.

**FOR STEP a (Examples 1 and 2 hereafter):** The reaction by milling allows the following improvements compared to manufacturing process in solution:
1) to reduce the reaction times to 2h or 3h (examples 1 and 2 respectively), compared to the manufacturing process in solution, which requires 17 h:
2) to avoid the use of (toxic) organic bases (e.g., triethylamine), which are herein replaced by the inorganic (non-toxic) base K₂CO₃, leading to an improved eco footprint of the process;
3) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

According to a particular embodiment, the batch, continuous or semi-continuous mechanochemical process of the invention enables the preparation of a compound of formula (III): 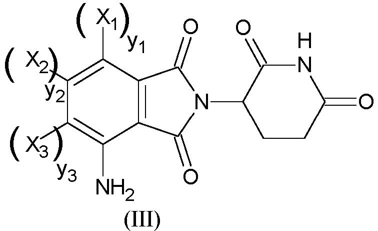 in particular of formula 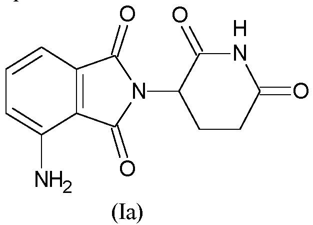 wherein X₁, X₂, X₃, y₁, y₂ and y₃ have the meaning defined above, said process comprising:
a) a cycloimidation step, wherein 3-aminopiperidine-2,6-dione hydrochloride is used as starting material, in the presence of
   a carboxylic function bearer chosen among 3-nitrophthalic acid or 3-nitrophthalic anhydride,
   a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-Ethyl-3-(3-dimethylaminopropyl)carbodiimide (EDC)
   and optionally a base, possibly generated *in situ* by said coupling agent,
      optionally at least one liquid additive is used, in particular dimethyl sulfoxide or dimethyl isosorbide, or a mixture of thereof,
   and said mechanical forces are generated by RAM and said cycloimidation step is carried out in LAG conditions, to obtain a crude product containing a compound of formula (IV), 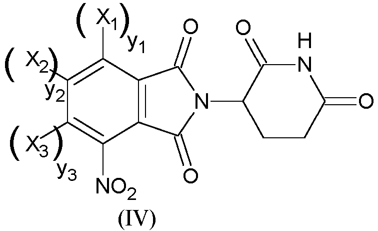 wherein X₁, X₂, X₃, y₁, y₂ and y₃ have the meaning defined above,

   wherein in the step a), the LAG conditions are defined by the parameter η that is greater than 0 µL/mg and less than or equal to 1 µL/mg, more preferably is comprised from about 0.2 µL/mg to 0.8 µL/mg, more preferably 0.5 µL/mg,
   said process comprising
b) a step of mechanochemical reduction of the above-mentioned compound of formula (IV), said mechanical forces being generated by RAM, to obtain a crude product containing said compound of formula (III),
wherein in the step b), the LAG conditions are defined by the parameter η that is greater than 0 µL/mg and less than or equal to 1 µL/mg, more preferably is comprised from about 0.60 µL/mg to 0.90 µL/mg, more preferably 0.75 µL/mg.

According to a particular embodiment of the batch, continuous or semi-continuous mechanochemical process of the invention, the additive is liquid and is dimethyl sulfoxide.

According to a particular embodiment of the batch, continuous or semi-continuous mechanochemical process of the invention, the additive is liquid and is dimethyl isosorbide (DMI).

According to a particular embodiment of the batch, continuous or semi-continuous mechanochemical process of the invention, the step a) is carried out in the absence of a base.

According to a particular embodiment of the batch, continuous or semi-continuous mechanochemical process of the invention, in the step b) in LAG conditions, the said at least one additive is liquid and is a mixture of water and an alcohol, in particular ethanol.

According to a particular embodiment of the batch, continuous or semi-continuous mechanochemical process of the invention, in step a), the g force is comprised from 2 g to 100 g, in particular 90 g.

According to a particular embodiment of the batch, continuous or semi-continuous mechanochemical process of the invention, in step b), the g force is comprised from 2 g to 100 g, in particular 90 g.

According to a particular embodiment of the batch, continuous or semi-continuous mechanochemical process of the invention, the compound of formula (II) is recovered from the crude product containing it, by a washing with water, a filtration and a removal of said water.

According to a particular embodiment of the batch, continuous or semi-continuous mechanochemical process of the invention, the step a) and/or the step b) are performed:
- under cooling at a temperature from -180°C to 24°C or
- heating at a temperature from 25°C to 150°C

According to a particular embodiment of the batch, continuous or semi-continuous mechanochemical process of the invention, the compound of formula (I) is recovered from the crude product containing it, by a washing with boiling solvent, in particular acetone, a filtration and an evaporation of said acetone.

According to a particular embodiment of the batch, continuous or semi-continuous mechanochemical process of the invention, the compound of formula (III) is recovered from the crude product containing it, by refluxing it with an hydrochloric acid solution, a filtration, a washing with a solution of NaHCO₃ and distilled water, a drying, and an evaporation of said water.

According to a particular embodiment of the batch, continuous or semi-continuous mechanochemical process of the invention, the compound of formula (III) is recovered from the crude product containing it, by an introduction of gaseous hydrochloric acid, and a washing with NaHCO₃ or an introduction of gaseous NH₃.

As a matter of example and in a non-limiting manner, the step a) can be carried out with a RAM operating at 20-22°C, with 3-nitrophthalic anhydride as the carboxylic acid bearer, 3-aminopiperidine-2,6-dione hydrochloride as the starting material and dimethyl sulfoxide as liquid additive in LAG conditions.

As a matter of example and in a non-limiting manner, the step a) can be carried out with a RAM operating at 20-22°C, with 3-nitrophthalic acid as the carboxylic acid bearer, 3-aminopiperidine-2,6-dione hydrochloride as the starting material and DMI (1,3-dimethyl-2-imidazolidinone) as liquid additive in LAG conditions.

As a matter of example and in a non-limiting manner, the step b) can be carried out with a RAM operating at 20-22°C, with 2-(2,6-dioxopiperidin-3-yl)-4-nitroisoindoline-1,3-dione as compound of Formula (IV), sodium dithionite, water and ethanol as liquid additive in LAG conditions.

**FOR STEP a (Examples 7-10 hereafter):** The reaction by resonant acoustic mixing (RAM) allows the following improvements compared to manufacturing process in solution:
1) to reduce the reaction times to 3h compared to the manufacturing process in solution, which requires 17 h:
2) to avoid the use of (toxic) organic bases (e.g., triethylamine), which are herein replaced by the inorganic (non-toxic) bases (e.g., K₂CO₃, K₃PO₄) leading to an improved eco footprint of the process;
3) to avoid the heating at 80°C, the reaction occurring at room temperature, making the process less-energy demanding;
4) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

**FOR STEP b (Example 11 hereafter):** The reduction reaction by resonant acoustic mixing (RAM) allows the following improvements compared to manufacturing process in solution:
1) to reduce of the reaction times to 1h compared to the manufacturing process in solution, which requires 6.5 h:
2) to circumvent the use of precious, toxic and costly raw materials such as metal-based catalysts, for which shortage is also an issue (e.g., palladium catalyst), also making the recovery of the final product cumbersome (for the catalyst separation) and generating toxic waste. The reduction by RAM is 'metal-free', make use cheap reagent (e.g. Na₂S₂O₄), generate not-harmful waste, leading to an improved eco-footprint of the process;
3) to avoid the heating at 50-60°C, the reaction occurring at room temperature, making the process less-energy demanding;
4) to avoid the use of harmful (and high boiling temperature) solvents such as 1,4-dioxane.

According to a particular embodiment, the continuous mechanochemical process of the invention enables the preparation of a compound of formula (III): 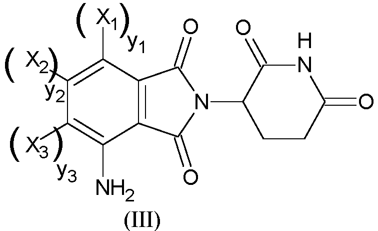 in particular of formula 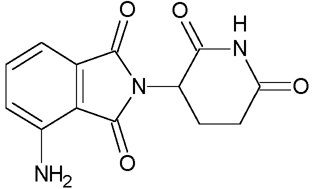 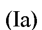 wherein X₁, X₂, X₃, y₁, y₂ and y₃ have the meaning defined above, said process comprising:
a) a cycloimidation step, wherein 3-aminopiperidine-2,6-dione hydrochloride is used as starting material, in the presence of
   a carboxylic function bearer which is 3-nitrophthalic acid or 3-nitrophthalic anhydride, and optionally at least one additive is used, in particular dimethyl sulfoxide or dimethyl isosorbide, or a mixture of thereof,
   and optionally a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-Ethyl-3-(3-dimethylaminopropyl)carbodiimide (EDC),
   and optionally a base, possibly generated *in situ* by said coupling agent, and,
   said cycloimidation step is carried out under neat grinding, to obtain a crude product containing a compound of formula (IV), 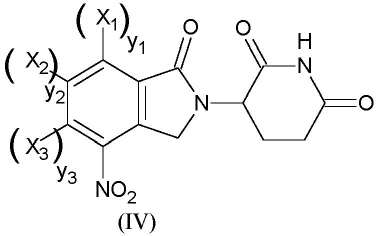 wherein X₁, X₂, X₃, y₁, y₂ and y₃ have the meaning defined above,
   said process comprising
b) a step of mechanochemical reduction of the above-mentioned compound of formula (IV), to obtain a crude product containing said compound of formula (III),
wherein in the step b), the LAG conditions are defined by the parameter η that is greater than 0 mL/g and less than or equal to 1 mL/g, more preferably is comprised from about 0.50 mL/g to 1 mL/g, more preferably 0.75 mL/g,

According to a particular embodiment of the continuous mechanochemical process of the invention, in step a) the mechanical forces are generated by twin screw extrusion.

According to a particular embodiment of the continuous mechanochemical process of the invention, the step a) is carried out in the presence of K₂CO₃ as a base.

In this embodiment, the role K₂CO₃ is to avoid corrosion. The process has a reduced environmental footprint by avoiding base-derived waste from a supplementary base implied in the mechanism of the step a).

According to a particular embodiment of the continuous mechanochemical process of the invention, in the step b) in LAG conditions, the said at least one additive is liquid and is a mixture of water and an alcohol, in particular ethanol.

According to a particular embodiment of the continuous mechanochemical process of the invention, in step b) the mechanical forces are generated by twin screw extrusion.

According to a particular embodiment of the continuous mechanochemical process of the invention, the compound of formula (III) is recovered from the crude product containing it, by refluxing it with a hydrochloric acid solution, a filtration, optionally a washing with a solution of NaHCO₃ and distilled water, and a drying.

As a matter of example and in a non-limiting manner and according to a particular embodiment of the continuous mechanochemical process of the invention, in step a), the milling device is twin screw extruder and the milling speed value is chosen from 1 to 2000 rpm or from 50 to 1000 rpm, in particular 60 rpm.

As a matter of example and in a non-limiting manner and according to a particular embodiment of the continuous mechanochemical process of the invention, in step b), the milling device is twin screw extruder and the milling speed value is chosen from 1 to 2000 rpm or from 50 to 1000 rpm, in particular 60 rpm.

According to a particular embodiment of the mechanochemical continuous process of the invention, the compound of formula (III) is recovered from the crude product containing it by introducing gaseous hydrochloric acid into the extrusion barrel, optionally followed by washing with a solution of NaHCO₃, or by introducing gaseous NH₃, and subsequently precipitating in water.

In this embodiment, the extruder or batch milling device (such as ball-milling jars equipped with appropriate gas valves) is constructed from materials resistant to gaseous hydrochloric acid. The introduction of gaseous hydrochloric acid serves to cleave side-products potentially formed during the mechanochemical reduction step.

Depending on the specific compound processed, the introduction of gaseous hydrochloric acid may result either in the formation of an ammonium salt or directly yield the free-base form of the compound of formula (III). Consequently, if the ammonium salt is formed, further mechanochemical treatment involving mixing with NaHCO₃ or exposure to gaseous NH₃ may be carried out to convert the salt into the desired free-base form of the compound. This optional treatment enhances process flexibility and contributes positively to the overall environmental footprint by reducing the usage of aqueous media.

As a matter of example and in a non-limiting manner, the step a) can be carried out with a twin screw extruder operating at 180°C, with 3-nitrophthalic anhydride as the carboxylic acid bearer and 3-aminopiperidine-2,6-dione hydrochloride as the starting material under neat grinding.

As a matter of example and in a non-limiting manner, the step b) can be carried out with a twin screw extruder operating at 70°C, with 2-(2,6-dioxopiperidin-3-yl)-4-nitroisoindoline-1,3-dione as compound of Formula (IV), sodium dithionite, water and ethanol as liquid additive in LAG conditions.

**FOR STEP a (Example 5 hereafter):** The reaction by TSE allows the following improvements compared to manufacturing process in solution:
1) to avoid the use of (toxic) organic bases (e.g., triethylamine), which are herein replaced by the inorganic (non-toxic) base (*e.g*., K₂CO₃) leading to an improved eco footprint of the process;
2) to avoid the use of harmful (and high boiling temperature) solvents such as DMF, also responsible for the formation of toxic impurities such as nitrosamines, usually formed during the manufacturing process in solution, especially upon heating. Nitrosamines are harmful and toxic compounds posing risks for the patient health.

According to a particular embodiment, the batch mechanochemical process of the invention enables the preparation of a compound of formula (VII): 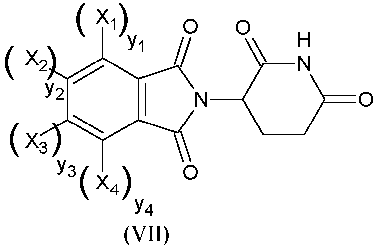 in particular of formula 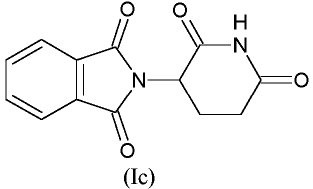 wherein:
- X₁, X₂, X₃ and X₄, identical or different, are chosen among: (C₁-C₅)alkyl group, (C₁-C₅)alkoxy group, halogen atom, trifluoromethyl group, hydroxy group, (H or C₁-C₅)acyl group, a 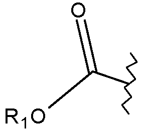 group, or a 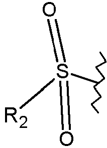 group
   wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
   and R₂ is chosen among: hydroxy group, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
a) a cycloimidation step wherein 3-aminopiperidine-2,6-dione hydrochloride is used as starting material, in the presence of
   a carboxylic function bearer chosen among phthalic acid or phthalic anhydride,
   a coupling agent chosen among carbonyldiimidazole (CDI), *N,N,N,N-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), in particular carbonyldiimidazole (CDI), 1-Ethyl-3-(3-dimethylaminopropyl)carbodiimide (EDC), and
   a base, possibly generated *in situ* by said coupling agent, and optionally at least one additive is used, in particular dimethyl sulfoxide,
   and said mechanical forces are generated by a milling device and said cycloimidation step is carried out under neat grinding, or with or without adjuvant, or in LAG conditions with or without adjuvant,
   to obtain a crude product containing said compound of formula (VIII),
   wherein when the step a) is in LAG conditions, the latter are defined by the parameter η that is greater than 0 µL/mg and less than or equal to 1 µL/mg, more preferably is comprised from about 0.05 µL/mg to 0.35 µL/mg, more preferably 0.1 µL/mg.

According to a particular embodiment of the batch, continuous or semi-continuous mechanochemical process of the invention, when the step a) is in LAG conditions, the additive is liquid and is dimethyl sulfoxide.

According to a particular embodiment of the batch, continuous or semi-continuous mechanochemical process of the invention, when the step a) is in LAG conditions, the additive is liquid and is chosen among dimethyl carbonate, ethyl acetate, dimethyl isosorbide, isopropylalcohol, acetonitrile, dimethyl formamide, tetrahydrofuran, cyrene, acetone, sulfolane, butan-2-one or a mixture of thereof. According to a particular embodiment of the batch, continuous or semi-continuous mechanochemical process of the invention, when the step a) is carried out in the presence of a solid adjuvant, said solid adjuvant being chosen among sodium chloride, magnesium sulfate or a mixture of thereof.

According to a particular embodiment of the batch, continuous or semi-continuous mechanochemical process of the invention, the step a) is carried out in the presence of CDI as a coupling agent and imidazole as a base, generated *in situ* from CDI.

According to a particular embodiment of the batch mechanochemical process of the invention, in the step a) the milling device is PBM.

As a matter of example and in a non-limiting manner and according to a particular embodiment of the batch mechanochemical process of the invention, in step a) carried out under neat grinding, the milling device is PBM, the milling speed value is chosen from 1 to 1100 rpm, in particular 850 rpm.

As a matter of example and in a non-limiting manner and according to a particular embodiment of the batch mechanochemical process of the invention, in step a) carried out in LAG conditions, the milling device is PBM and the milling speed value is chosen from 1 to 1100 rpm, in particular 450 rpm.

As a matter of example and in a non-limiting manner, the step a) can be carried out with a planetary ball-mill extruder operating, with phthalic anhydride as the carboxylic acid bearer, 3-aminopiperidine-2,6-dione hydrochloride as the starting material and CDI as the coupling agent under neat grinding. As a matter of example and in a non-limiting manner, the step a) can be carried out with a planetary ball-mill extruder operating, with phthalic anhydride as the carboxylic acid bearer, 3-aminopiperidine-2,6-dione hydrochloride as the starting material, CDI as the coupling agent and DMSO as additive in LAG conditions.

According to a particular embodiment, the batch mechanochemical process of the invention enables the preparation of a compound of formula (VII): 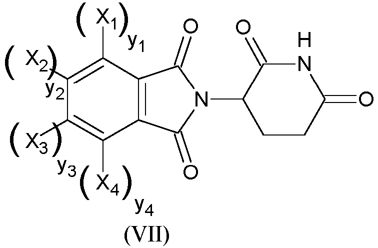 in particular of formula 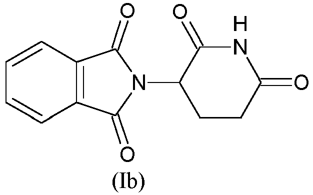 wherein:
- X₁, X₂, X₃ and X₄, identical or different, are chosen among: (C₁-C₅)alkyl group, (C₁-C₅)alkoxy group, halogen atom, trifluoromethyl group, hydroxy group, (H or C₁-C₅)acyl group, a 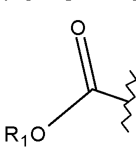 group, or a 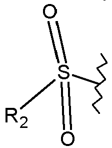 group
   wherein R₁ is chosen among: hydrogen atom, (C₁-C₅)alkyl group, or -NR₁ₐR_{1b} group,
   R₁ₐ and R_{1b}, identical or different, are chosen among: hydrogen atom or (C₁-C₅)alkyl group,
   and R₂ is chosen among: hydrogen atom, (C₁-C₅)alkoxy group, -NR₂ₐR_{2b},
   R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
a) a cycloimidation step, wherein L-glutamine is used as starting material, in the presence of a carboxylic function bearer which is phthalic anhydride,
   a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), in particular carbonyldiimidazole (CDI), 1-Ethyl-3-(3-dimethylaminopropyl)carbodiimide (EDC), and
   a base, possibly generated *in situ* by said coupling agent, and
      optionally at least one additive is used, in particular dimethyl sulfoxide,
   and said mechanical forces are generated by a milling device and said cycloimidation step is carried out under neat grinding, or with or without a solid adjuvant, or in LAG conditions, with or without a solid adjuvant,
   to obtain a crude product containing said compound of formula (VII),
wherein when the step a) is in LAG conditions, the latter are defined by the parameter η that is greater than 0 µL/mg and less than or equal to 1 µL/mg, more preferably is comprised from about 0.05 µL/mg to 0.35 µL/mg, more preferably 0.2 µL/mg.

According to a particular embodiment of the batch, continuous or semi-continuous mechanochemical process of the invention, when the step a) is in LAG conditions, the additive is liquid and is dimethyl sulfoxide.

According to a particular embodiment of the batch, continuous or semi-continuous mechanochemical process of the invention, when the step a) is in LAG conditions, the additive is liquid and is chosen among dimethyl carbonate, ethyl acetate, dimethyl isosorbide, isopropylalcohol, acetonitrile, dimethyl formamide, tetrahydrofuran, cyrene, acetone, sulfolane, butan-2-one or a mixture of thereof.

According to a particular embodiment of the batch, continuous or semi-continuous mechanochemical process of the invention, when the step a) is carried out in the presence of a solid adjuvant, said solid adjuvant being chosen among sodium chloride, magnesium sulfate or a mixture of thereof.

According to a particular embodiment of the batch, continuous or semi-continuous mechanochemical process of the invention, the step a) is carried out in the presence of CDI as coupling agent, and imidazole as a base, generated *in situ* from CDI.

According to a particular embodiment of the batch mechanochemical process of the invention, in the step a) the milling device is PBM.

As a matter of example and in a non-limiting manner and according to a particular embodiment of the batch mechanochemical process of the invention, in step a), the milling device is PBM and the milling speed value is chosen from 1 to 1100 rpm, in particular 850 rpm.

As a matter of example and in a non-limiting manner, the step a) can be carried out with a planetary ball-mill extruder operating at 20-22°C, with phthalic anhydride as the carboxylic acid bearer, L-glutamine as the starting material and CDI as the coupling agent under neat grinding.

As a matter of example and in a non-limiting manner, the step a) can be carried out with a planetary ball-mill extruder operating at 20-22°C, with phthalic anhydride as the carboxylic acid bearer, L-glutamine as the starting material, CDI as the coupling agent and DMSO (dimethylsulfoxide) as additive in LAG conditions.

### LIST OF FIGURES

**Figure 1****.** ¹H NMR spectrum of compound P1, signal intensity (arbitrary unit) in abscissae as a function of the chemical shift in the ordinate (in ppm)
**Figure 2****.** ¹³C NMR spectrum of compound P1, signal intensity (arbitrary unit) in abscissae as a function of the chemical shift in the ordinate (in ppm)
**Figure 3****.** ¹H NMR spectrum of compound (Ia), signal intensity (arbitrary unit) in abscissae as a function of the chemical shift in the ordinate (in ppm)
**Figure 4****.** ¹³C NMR spectrum of compound (Ia), signal intensity (arbitrary unit) in abscissae as a function of the chemical shift in the ordinate (in ppm)
**Figure 5** corresponds to the detailed configuration of a ZE9 twin-screw extruder in step a) of preparation of pomalidomide (to obtain P1), the numbers displayed are millimeters, as follows:
   1. **LD 25:1 twin screw ((A) ; OrderNr: C04094):** A primary twin screw element with a length of 78.5 mm and a pitch of 13.5 mm, ensuring efficient conveying of materials.
   2. **Mixing screw elements OrderNr: ((B) ; C04871):** A mixing element with a length of 9 mm and a pitch of 9 mm, designed to ensure thorough mixing of the reactants.
   3. **Kneading blocks OrderNr: ((C,D) ; C03291):** Two kneading segments with a length of 3 mm, 90° phase angle, to provide initial intensive shear.
   4. **Concave element ((E) ; OrderNr: C03278** Further concave element with a length of 9 mm and a pitch of 9 mm, placed to aid in the consistent conveyance and mixing of the materials.
   5. **Additional Kneading segment OrderNr: ((F) ; C03291):** Further kneading segment with same dimensions as mentioned above.
   6. **Additional concave elements ((G, H, I, J, K, L, M, N) ; OrderNr: C03930** Further concave element with a length of 13.5 mm and a pitch of 9 mm, placed to aid in the consistent conveyance and mixing of the materials towards the end of the process.
   7. **End screws and segments OrderNr: ((O) ; C04872:)** End segment with a concave shape, positioned at the final stage to ensure thorough processing and discharge of the extrudate.

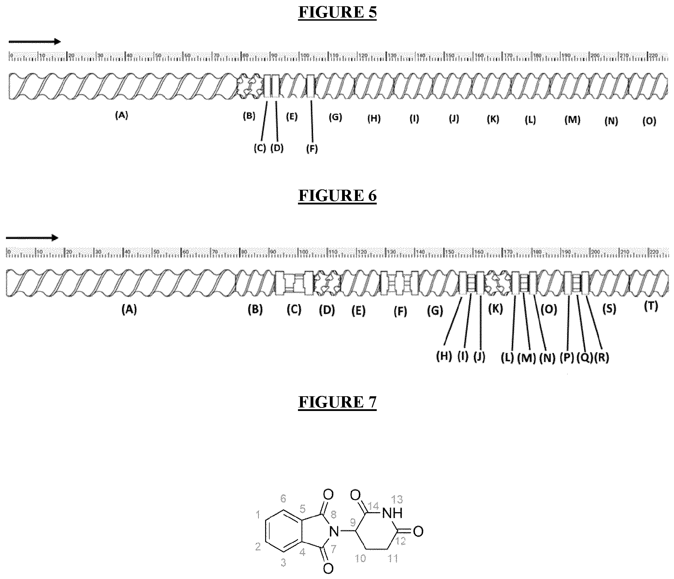

**Figure 6** corresponds to the detailed configuration of a ZE9 twin-screw extruder in step b) of preparation of pomalidomide, the numbers displayed are millimeters, as follows:
   1**. LD 25:1 twin screw ((A) ; OrderNr: C04094):** A primary twin screw element with a length of 78.5 mm and a pitch of 13.5 mm, ensuring efficient conveying of materials.
   2. **Concave screw elements ((B), (E), (G), (S) ; OrderNr: C03930):** Multiple concave elements with a length of 13.5 mm and a pitch of 9 mm, placed at various intervals to aid in consistent material transport and initial mixing.
   3. **Kneading blocks:**
      ∘ **OrderNr: ((C) ; C03935):** A kneading block with a length of 13.5 mm, 60° phase angle, and 4 kneading segments, positioned to provide initial intensive shear.
      ∘ **OrderNr: ((F) ; C09042):** Another kneading block with a length of 13.5 mm, 90° phase angle, and 5 kneading segments, enhancing the mixing and kneading process.
      ∘ **OrderNr: ((H), (I), (J), (L), (M), (N), (P, (Q), (R); C03291 and C03292:** Several shorter kneading elements with lengths of 3 mm, phase angles of 0° and 90°, respectively, strategically placed to continue the shearing and kneading process.
   4. **Mixing screw elements:**
      ∘ **OrderNr: ((D) ; C04871):** A mixing element with a length of 9 mm and a pitch of 9 mm, designed to ensure thorough mixing of the reactants.
      ∘ **OrderNr: ((K) ; C08930):** An inverted mixing element with a length of 9 mm and a pitch of 9 mm, creating backflow to enhance mixing efficiency.
   5. **Additional concave elements ((O) ; OrderNr: C03278 and C03930** : Further concave elements with the same dimensions as previously mentioned, placed to aid in the consistent conveyance and mixing of the materials towards the end of the process.
   6. **End screws and segments:**
      ∘ **OrderNr: C03938:** End screw elements with no pitch, ensuring complete discharge of the material from the extruder.
      ∘ **OrderNr: ((T) ; C04872:)** End segment with a concave shape, positioned at the final stage to ensure thorough processing and discharge of the extrudate.
**Figure 7****.** Scheme representing the thalidomide structure, and the number are corresponding to the number of carbon atoms in the signal of the ¹³C NMR of Example 13, Example 14 and Example 15

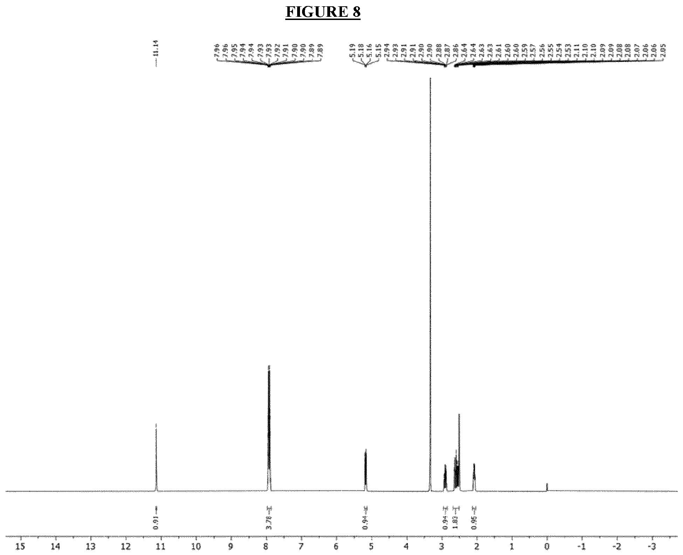

**Figure 8****.** ¹H NMR spectrum of compound (Ib), signal intensity (arbitrary unit) in abscissae as a function of the chemical shift in the ordinate (in ppm)

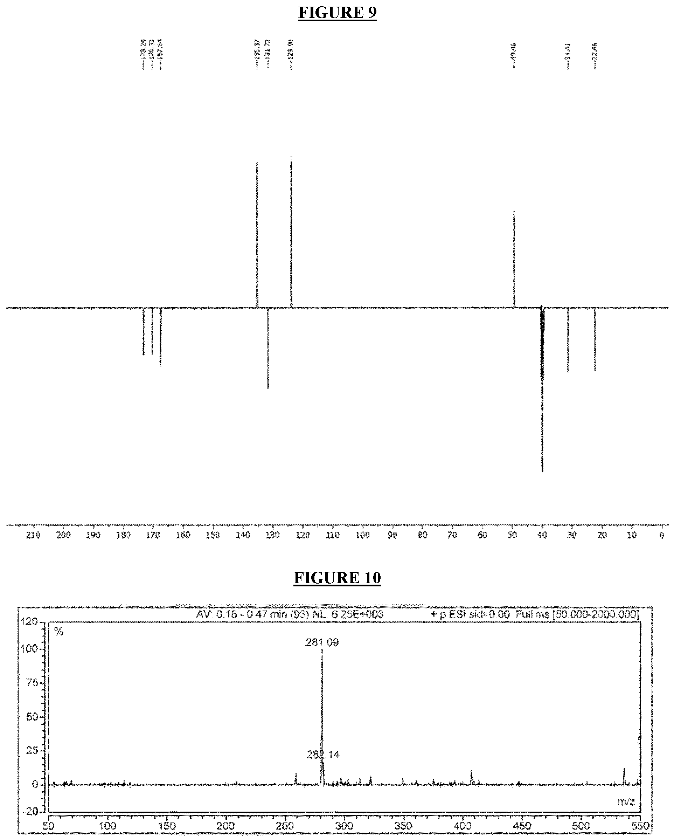

**Figure 9****.** ¹³C Attached-Proton-Test (APT) NMR spectrum of compound (Ib), signal intensity (arbitrary unit) in abscissae as a function of the chemical shift in the ordinate (in ppm)
**Figure 10****.** Electrospray Ionization Mass Spectrometry spectrum of compound (Ib), the mass to charge ratio as a function of the percentage of the chemical compound (in percentage)

### Example 1 - Step a) of preparation of pomalidomide under planetary ball milling (PBM)

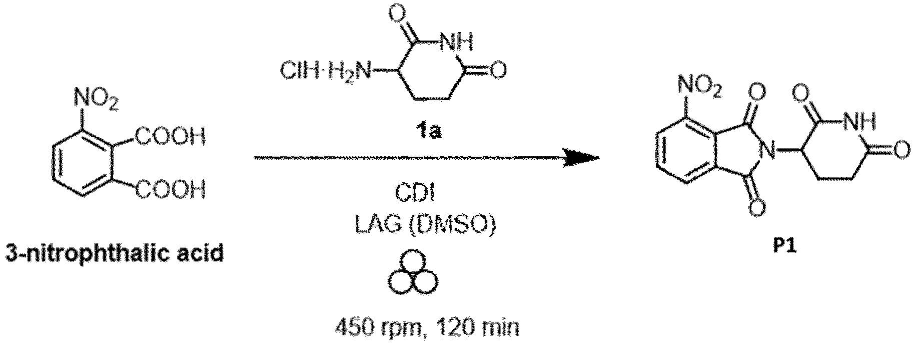

3-aminopiperidine-2,6-dione hydrochloride (1a) (329.2 mg, 2.0 mmol), 3-nitrophthalic acid (464.5 mg, 2.2 mmol, 1.1 eq), and carbonyldiimidazole (CDI) (745.9 mg, 4.6 mmol, 2.3 eq) were placed in a 20 mL ZrO₂ jar charged with 40 x 5 mm ZrO₂ balls. Then, liquid additive was added (DMSO, 308 µL, η = 0.2 µL/mg) and milled at 450 rpm for 120 min to obtain a reaction crude. To the resulting reaction crude, water (20 mL) was added to form a precipitate, and said precipitate was isolated by filtration to obtain a solid. The solid was washed with water (3 x 10 mL) and dried *in vacuo* in the presence of P₂O₅, to give 2-(2,6-Dioxopiperidin-3-yl)-4-nitroisoindoline-1,3-dione (P1) as a white solid (430.5 mg, 71% yield).

¹H NMR (400 MHz, DMSO-*d₆*) δ (ppm) 11.18 (s, 1H), 8.35 (d, ³J = 7.9 Hz, 1H), 8.24 (d, ³J = 7.3 Hz, 1H), 8.12 (t, ³J = 7.8 Hz, 1H), 5.21 (dd, ³J = 12.9, 5.4 Hz, 1H), 2.98 - 2.79 (m, 1H), 2.66-2.57 (m, 1H), 2.56 - 2.44 (m, 2H), 2.14 - 1.99 (m, 1H). ¹³C NMR (101 MHz, DMSO-*d₆*) δ (ppm) 172.71, 169.50, 165.17, 162.52, 144.43, 136.80, 133.00, 128.87, 127.30, 122.56, 49.44, 30.87, 21.74. HRMS ESI (+): m/z calculated for C₁₃H₁₀N₃O₆ [M+H]⁺ 304.0564, found 304.0561.

### Example 2 - Step a) of preparation of pomalidomide under vibrating ball milling (VBM)

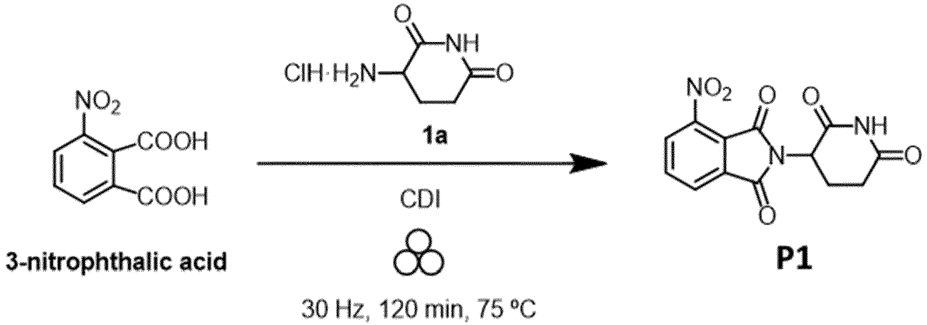

3-aminopiperidine-2,6-dione hydrochloride (**1a**) (246.9 mg, 1.5 mmol), 3-nitrophthalic acid (348.4 mg, 1.65 mmol, 1.1 eq), and CDI (559.4 mg, 3.45 mmol, 2.3 eq) were placed in a 10 mL hardened stainless-steel jar charged with 2x10 mm hardened stainless steel balls and milled at 30 Hz for 120 min maintaining the temperature at 75 °C, to obtain a reaction crude. To the resulting reaction crude, water (20 mL) was added to form a precipitate and said precipitate was isolated by filtration to obtain a solid. The solid was washed with water (3x10 mL) and dried *in vacuo* in the presence of P₂O₅, to give 2-(2,6-Dioxopiperidin-3-yl)-4-nitroisoindoline-1,3-dione (**P1**) as an off-white solid (374.5 mg, 1.23 mmol, 82% yield).

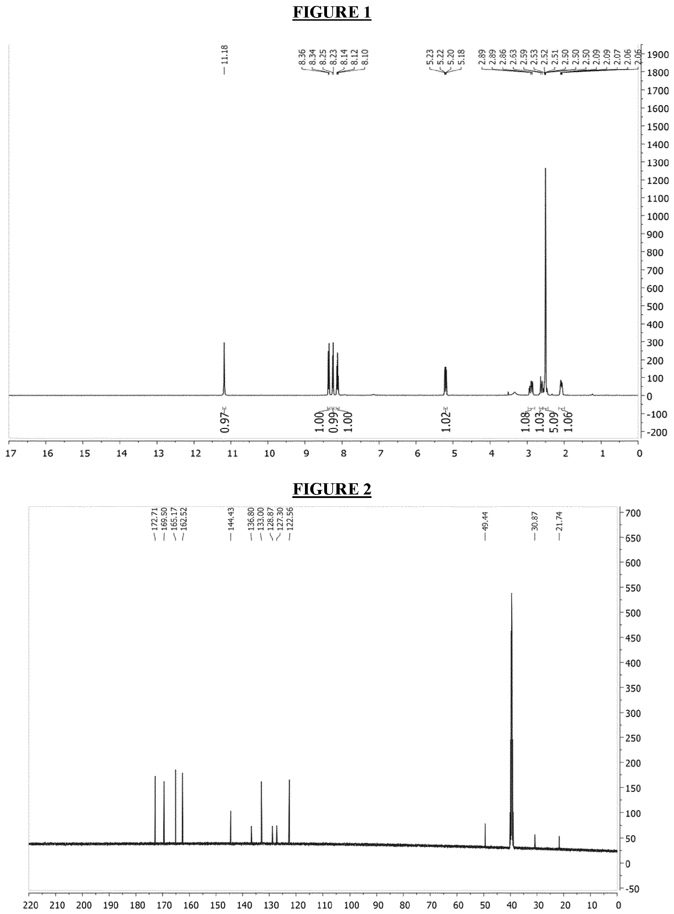

¹H NMR (400 MHz, DMSO-d₆, spectrum is on Figure 1) δ (ppm) 11.18 (s, 1H), 8.35 (d, ³J = 7.9 Hz, 1H), 8.24 (d, ³J = 7.3 Hz, 1H), 8.12 (t, ³J = 7.8 Hz, 1H), 5.21 (dd, ³J = 12.9, 5.4 Hz, 1H), 2.98 - 2.79 (m, 1H), 2.66-2.57 (m, 1H), 2.56 - 2.44 (m, 2H), 2.14 - 1.99 (m, 1H). ¹³C NMR (101 MHz, DMSO-d₆, spectrum is on Figure 2) δ(ppm) 172.71, 169.50, 165.17, 162.52, 144.43, 136.80, 133.00, 128.87, 127.30, 122.56, 49.44, 30.87, 21.74. HRMS ESI(+): m/z calculated for C₁₃H₁₀N₃O₆ [M+H]⁺ 304.0564, found 304.0561.

### Example 3 - Step b) of preparation of pomalidomide under vibrating ball milling (VBM)

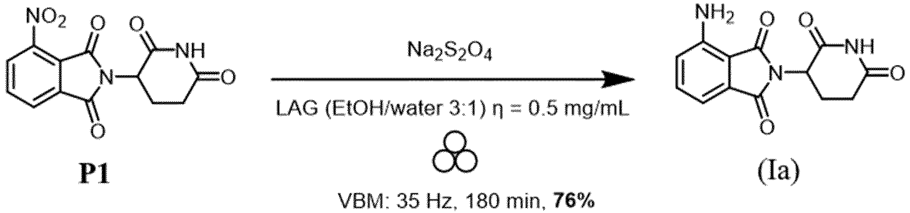

2-(2,6-Dioxopiperidin-3-yl)-4-nitroisoindoline-1,3-dione (**P1**) (333.6 mg, 1.1 mmol) and Na₂S₂O₄ (766.0 mg, 4.4 mmol, 4 eq) were placed in a 10 mL ZrO₂ jar charged with 1 x 10 mm ZrO₂ ball. Then, liquid additive was added (EtOH, 412 µL; H₂O, 137 µL, η = 0.5 µL/mg) and milled at 35 *Hz* for 180 min to obtain a reaction crude.

To the resulting reaction crude, boiling acetone (30 mL) was added and the mixture filtrated to obtain a solid residue. The solid residue was washed with additional boiling acetone (2x30 mL). The filtrate was collected, evaporated and dried to obtain (Ia) as a yellow solid (229.0 mg, 76% yield).

### Example 4 - Step b) of preparation of pomalidomide under planetary ball milling (PBM)

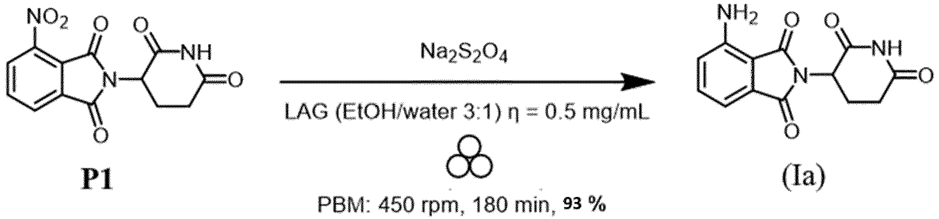

2-(2,6-Dioxopiperidin-3-yl)-4-nitroisoindoline-1,3-dione (**P1**) (3.154 g, 10.4 mmol) and Na₂S₂O₄ (7.243 g, 41.6 mmol, 4 eq) were placed in a 50 mL ZrO2 jar charged with 120 x 5 mm ZrO₂ balls. Then, liquid additive was added (EtOH : 3.8 mL; H₂O: 1,3 mL, η = 0.5 µL/mg) and milled at 450 rpm for 180 min to obtain a reaction crude.

To the resulting reaction crude, HCl 1 M (30 mL) was added and the mixture heated at 70°C for 20 min to obtain a resulting precipitate. The resulting precipitate was isolated by filtration, washed with NaHCO3 (2x20 mL), water (20 mL) and dried under reduced pressure to give (Ia) as a yellow solid (2.629 g, 93% yield).

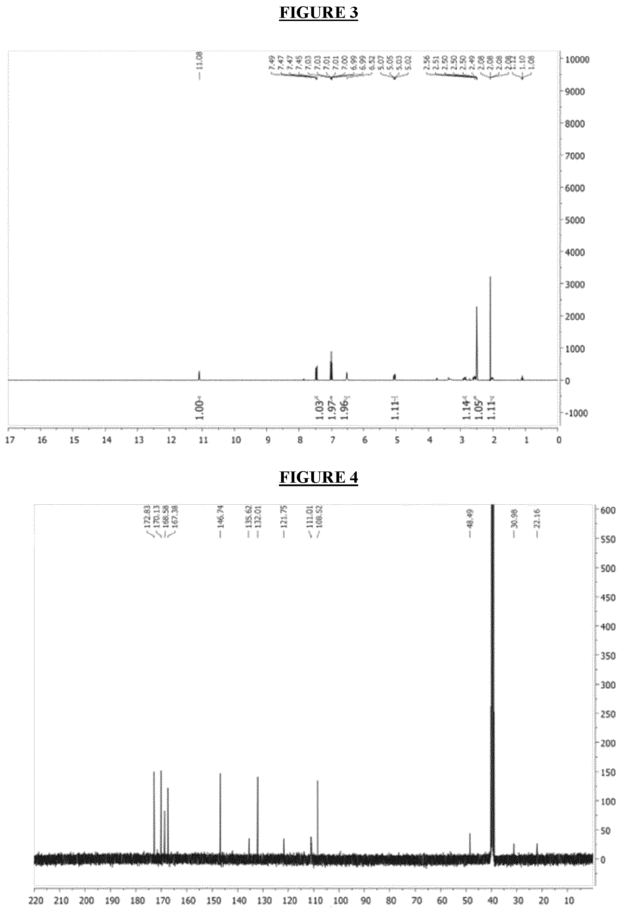

¹H NMR (400 MHz, DMSO-*d₆*, spectrum is on Figure 3) δ(ppm) 11.08 (s, 1H), 7.47 (dd, ³J = 8.5, 7.0 Hz, 1H), 7.02 (dd, ³J = 7.7 Hz, ⁴J = 0.8 Hz, 1 H), 7.00 (dd, ³J = 6.2 Hz, ⁴J = 0.7 Hz, 1 H), 6.52 (s, 2H, NH₂), 5.04 (dd, ³J = 12.7 Hz, 5.4 Hz), 2.94-2.82 (m, 1H), 2.62-2.52 (m, 2H), 2.07-1.97 (m, 1H) ). ¹³C NMR (101 MHz, DMSO-d₆, spectrum is on Figure 4) δ (ppm) 172.83, 170.13, 168.58, 167.38, 146.74, 135.62, 132.01, 121.75, 111.01, 108.52, 48.49, 30.98, 22.16. HRMS ESI (+): m/z calculated for C₁₃H₁₂N₃O₄ [M+H]⁺ 274.0822, found 274.0822.

### Example 5 - Step a) of preparation of pomalidomide under twin screw extrusion (TSE)

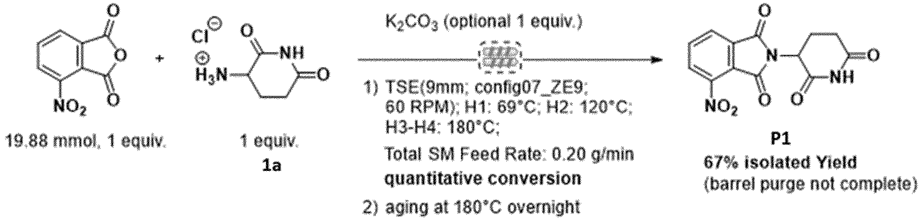

The reaction was performed by premixing the reactants-1 equivalent (19.88 mmol) each of 3-nitrophthalic anhydride, 3-aminopiperidine-2,6-dione hydrochloride, and K₂CO₃-using a mortar and pestle to obtain a homogeneous mixture. This homogeneous mixture was then introduced into the barrel of a ZE9 twin-screw extruder (Three-Tec; diameter: 9 mm; L/D = 25:1) using a gravimetric feeder set at 0.20 g/min. The screw profile used is described in Figure 5

The last two-barrel segments were preheated to 180 °C. Due to thermal transfer, the resulting temperature profile across the four-barrel zones, from inlet to outlet, was: 69 °C, 120 °C, 180 °C, and 180 °C. Once temperatures stabilized, extrusion was initiated at 60 RPM.

Upon completion of the addition of 3-nitrophthalic anhydride, 3-aminopiperidine-2,6-dione hydrochloride, and K₂CO₃, any remaining reaction mixture that was formed from the homogeneous mixture inside the barrel was flushed by feeding pure K₂CO₃ under identical extrusion conditions.

¹H NMR analysis of the crude product indicated full conversion of 3-nitrophthalic anhydride and 3-aminopiperidine-2,6-dione hydrochloride. However, partial conversion of mono-amidated intermediates to 2-(2,6-Dioxopiperidin-3-yl)-4-nitroisoindoline-1,3-dione (**P1**) was observed. To complete the reaction, the crude extrudate was aged overnight at 180 °C in an oven to form a solid crude.

The solid crude was then transferred into a glass frit and washed with distilled water until the filtrate became colorless (5 × 25 mL followed by 3 × 10 mL) to form a wet solid. The wet solid (59 wt% humidity) was subsequently dried in a desiccator containing P₂O₅, yielding pure **P1** in 67% yield.

¹H NMR (400 MHz, DMSO) δ 11.17 (s, 1H), 8.35 (dd, *J =* 8.1, 1.1 Hz, 1H), 8.24 (dd, *J =* 7.5, 1.0 Hz, 1H), 8.12 (t, *J =* 7.8 Hz, 1H), 5.20 (dd, *J =* 12.9, 5.4 Hz, 1H), 2.88 (ddd, *J =* 17.1, 13.9, 5.5 Hz, 1H), 2.61 (ddd, *J =* 17.0, 4.4, 2.3 Hz, 1H), 2.57 - 2.44 (m, 1H), 2.13 - 2.01 (m, 1H).

### Example 6 - Step b) of preparation of pomalidomide under twin screw extrusion (TSE)

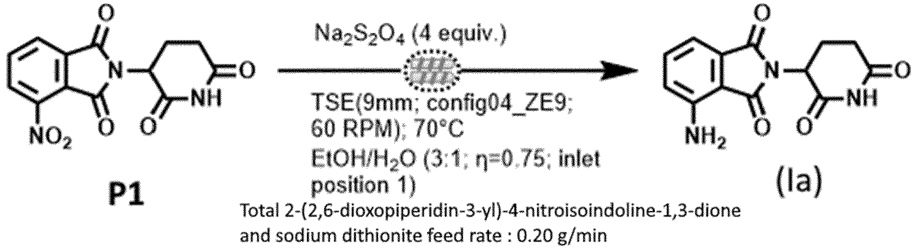

Solid 2-(2,6-dioxopiperidin-3-yl)-4-nitroisoindoline-1,3-dione (97wt% pure) and sodium dithionite (85 wt% pure, 4.00 equiv.) were homogenized into a mixture within a mortar and pestle and then transferred inside one feeder (total mass of the mixture is 10.15g). The screw profile used is described in Figure 6. Subsequently the 4 segments of the ZE9 barrel (Three-Tec, diameter: 9mm; L/D = 25:1) were heated at 70°C. Once temperature reached and stabilized extrusion started at 60 RPM. Solid mixture was fed at approximately 0.20 g/min while EtOH/H₂O mixture (3:1 volume ratio) serving as liquid additives in LAG conditions entered at 0.15 mL/min (η = 0.75 mL/g, liquid inlet located at 34.5 mm from the solid addition port or 73 mm from the beginning of the screw). When the addition of the solid mixture was finished, urea was filled into the feeder and used to flush out the remaining reaction crude from the barrel. The wet extrudate was further slurried with HCl 1M (3 mL/g of crude), the slurry refluxed for 20min, filtered on a glass frit (porosity n°4) to obtain a cake, rinsed with a satured solution of NaHCO₃ (15 mL) and with distilled water (2 x 10 mL) and dried (air dried and then in a vacuum oven at 40°C) to give (Ia) as a yellow powder (2.2915 g, 93.63 % yield).

Additional data:
1. During the workup, the step of rinsing using NaHCO₃ is optional, instead distilled water can be used to rinse the cake until the pH becomes neutral but it required much more water than NaHCO₃.
2. If traces of 2-(2,6-dioxopiperidin-3-yl)-4-nitroisoindoline-1,3-dione or sodium dithionite are present, another wash with acetone can get rid of them
3. An alternative workup can be the following: The wet extrudate was further slurred with HCl 1M (3 mL/g of crude), the slurry refluxed for 20 min, filtered on a glass frit (porosity n°4), rinsed with acetone (2 x 10 mL) and dried (air dried and then in a vacuum oven at 40°C) to give (Ia) as a yellow powder.

### Example 7 - First example of step a) of preparation of pomalidomide under resonant acoustic mixing (RAM)

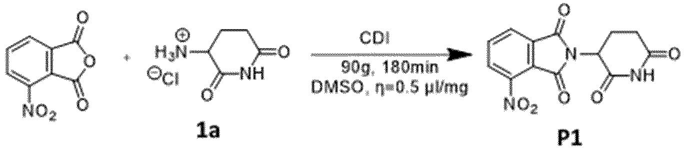

3-aminopiperidine-2,6-dione hydrochloride (658.6 mg, 3.9 mmol), 3-nitrophthalic anhydride (500 mg, 2.59 mmol), and CDI (755.7 mg, 4.7 mmol) and DMSO (957.2 µl, n=0.5 µl/mg) were mixed in a 7 ml glass in a LabRAM II Resodyn mixer at 90 g for 180 minutes to obtain a crude mixture. When the mixing was finished a lot of built-up pressure was released upon opening the vial leading to some loss of the crude mixture. The resulting crude mixture was transferred to a 100 mL beaker and distilled water (50 mL) was added to form a faint blue precipitate. Then the faint blue precipitate was filtered under vacuum to obtain a precipitate, and said precipitate was washed further with 50ml of distilled water and dried in vacuo in the presence of P₂O₅, to give 2-(2,6-Dioxopiperidin-3-yl)-4-nitroisoindoline-1,3-dione (P1) as a faint blue solid (501.5 mg, 64% yield).

¹H NMR (400 MHz, DMSO-d₆) δ (ppm) 11.18 (s, 2H), 8.35 (dd, *J =* 8.1, 0.9 Hz, 2H), 8.24 (dd, *J =* 7.5, 0.9 Hz, 2H), 8.12 (dd, *J =* 8.0, 7.6 Hz, 2H), 5.21 (dd, *J =* 12.9, 5.4 Hz, 2H), 2.89 (ddd, *J =* 17.2, 13.9, 5.4 Hz, 2H), 2.69 - 2.52 (m, 3H), 2.52 - 2.40 (m, 3H).

¹³C NMR (101 MHz, DMSO-*d₆*) δ (ppm) 172.83, 170.13, 168.58, 167.38, 146.74, 135.62, 132.01, 121.75, 111.01, 108.52, 48.49, 30.98, 22.16.

HRMS ESI(+): m/z calculated for C₁₃H₁₂N₃O₄ [M+H]⁺ 274.0822, found 274.0822.

### Example 8 - Second example of step a) of preparation of pomalidomide under resonant acoustic mixing (RAM)

3-aminopiperidine-2,6-dione hydrochloride (263.5 mg, 1.6 mmol), 3-nitrophthalic anhydride (250 mg, 1.3 mmol), and CDI (327.4 mg, 2.01 mmol) and DMI (420.5 µl, n=0.5 µl/mg) were mixed in a 4 ml glass vial in a LabRAM II Resodyn mixer at 90 g for 180 minutes to obtain a crude mixture. The crude mixture was transferred to a 100 mL beaker and distilled water (50 mL) was added to form a faint blue precipitate. Then the faint blue precipitate was filtered under vacuum to form a precipitate and said precipitate was washed further with 50ml of distilled water and dried in vacuo in the presence of P₂O₅, to give **P1** as a white solid (53 mg, 15% yield).

### Example 9 - Third example of step a) of preparation of pomalidomide under resonant acoustic mixing (RAM)

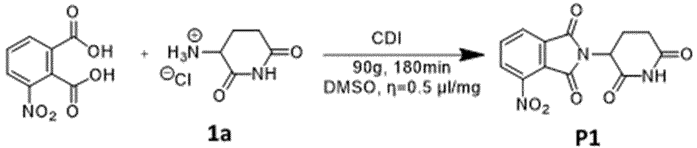

3-aminopiperidine-2,6-dione hydrochloride (481.9 mg, 2.9 mmol), 3-nitrophthalic acid (400 mg, 1.9 mmol), and CDI (706.6 mg, 4.4 mmol) and DMSO (794.2 µl, n=0.5 µl/mg) were mixed in a 7 mL glass vial in a LabRAM II Resodyn mixer at 90g for 180 minutes to obtain a crude mixture. When the mixing was finished a lot of built-up pressure was released upon opening the vial leading to some loss of the crude mixture. Said crude mixture was transferred to a 100ml beaker and distilled water (50 mL) was added and a white precipitate was formed. Then the white precipitate was filtered under vacuum to obtain a solid, and said solid was washed further with 50ml of distilled water and dried in vacuo in the presence of P₂O₅, to give **P1** as a white solid (301.2 mg, 52% yield).

¹HNMR (400 MHz, DMSO-*d₆*) δ (ppm) 11.19 (s, 1H), 8.37 (dd, *J* = 8.1, 0.8 Hz, 1H), 8.26 (dd, *J* = 7.5, 0.8 Hz, 1H), 8.18 - 8.09 (m, 1H), 5.22 (dd, *J* = 12.9, 5.4 Hz, 1H), 2.90 (ddd, *J =* 17.3, 13.9, 5.5 Hz, 1H), 2.60 (dd, *J* = 22.5, 10.8 Hz, 2H), 2.18 - 2.04 (m, 1H).

¹³C NMR (101 MHz, DMSO-*d₆*) δ (ppm) 173.20, 169.98, 165.65, 162.99, 144.90, 137.26, 133.47, 129.34, 127.77, 123.03, 121.31, 49.92, 31.34, 22.21.

HRMS ESI (+): m/z calculated for C₁₃H₁₂N₃O₄ [M+H]⁺ 274.0822, found 274.0822.

### Example 10 - Fourth example of step a) of preparation of pomalidomide under resonant acoustic mixing (RAM)

3-aminopiperidine-2,6-dione hydrochloride (192.8 mg, 1.14 mmol), 3-nitrophthalic acid (200 mg, 0.95 mmol), and CDI (353.3 mg, 2.2 mmol) and DMI (373 µl, n=0.5 µl/mg) were mixed in a 4 mL glass vial in a LabRAM II Resodyn mixer at 90g for 180 minutes to obtain a crude mixture. Said crude mixture was transferred to a 100ml beaker and distilled water (50 mL) was added and a white precipitate was formed. Then the white precipitate was filtered under vacuum to form a solid, and the resulting solid was washed further with 50ml of distilled water and dried in vacuo in the presence of P₂O₅, to give **P1** as a white solid (143 mg, 55% yield).

### Example 11 - First example of step b) of preparation of pomalidomide under resonant acoustic mixing (RAM)

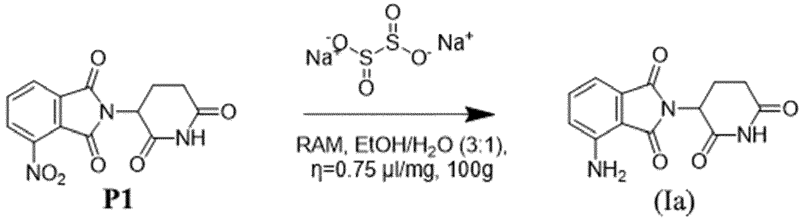

700mg (2.31mmol, 1eq) of 2-(2,6-Dioxopiperidin-3-yl)-4-nitroisoindoline-1,3-dione and 1.6g of sodium dithionite (9.2mmol, 4eq) were added in a 5 mL PTFE (polytetrafluoroethylene) jar. Then EtOH (1.3 mL) and water (433 µL) were added in a ratio of 3:1 and of a total η value of 0.75 µL/mg. Then the contents of the vial was mixed in a LabRAM II Resodyn mixer at 100 *g* for 60 minutes to form a crude mixture. After the reaction, the crude mixture was transferred into a round bottom flask and 100 mL acetone were added to form a solution. The solution was heated until boiling and then filtered while hot to obtain a filtrate. The filtrate was collected evaporated and dried over P₂O₅ to give (Ia) as bright yellow solid (518.7 mg, 82%).

¹H NMR (400 MHz, DMSO-d₆) δ (ppm) 11.10 (s, 1H), 7.48 (dd, *J* = 8.5, 7.0 Hz, 1H), 7.08 - 6.97 (m, 2H), 6.54 (s, 2H), 5.06 (dd, *J =* 12.9, 5.4 Hz, 1H), 2.90 (ddd, *J =* 17.5, 14.2, 5.5 Hz, 1H), 2.59 (dd, *J* = 19.1, 5.9 Hz, 1H), 2.07 - 1.86 (m, 1H).

¹³C NMR (101 MHz, DMSO-d₆) δ (ppm) 173.3, 170.6, 169.06, 167.85, 147.21, 135.95, 132.48,122.2, 111.46, 109.01, 48.95, 31.45, 22.71.

HRMS ESI (+); m/z [M+H]⁺: Calcd. for C₁₃H₁₂N₃O₄, 274.0827; found 274.0822

### Example 12 - Second example of step b) of preparation of pomalidomide under resonant acoustic mixing (RAM)

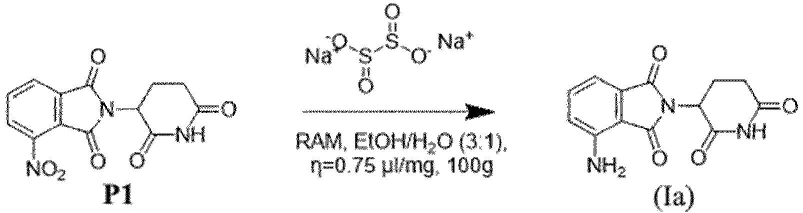

1000mg (3.3mmol, 1eq) of 2-(2,6-dioxopiperidin-3-yl)-4-nitroisoindoline-1,3-dione and 2.3g of sodium dithionite (13.2 mmol, 4 eq) were added in a 15 mL PTFE jar. Then EtOH 1.85 mL and water 618 µL were added in a ratio of 3: 1 and of a total η value of 0.75 µL/mg. Then the contents of the PTFE jar were mixed in a LabRAM II Resodyn mixer at 100 g for 60 minutes to obtain a mixture. After the reaction, the mixture was transferred into a 50 mL round bottom flask and 20 mL of 1M HCl were added to obtain a solution. The solution was heated at 80°C for 30 minutes and then filtered under vacuum to obtain a precipitate. The precipitate was washed with 50 mL NaHCO₃ saturated solution and 20 mL of distilled water to obtain a washed precipitate. Then the washed precipitate was collected and dried over P₂O₅ to give (Ia) as yellow solid (775.2 mg, 86%).

### Example 13 - First example of preparation of thalidomide under planetary ball-milling (PBM)

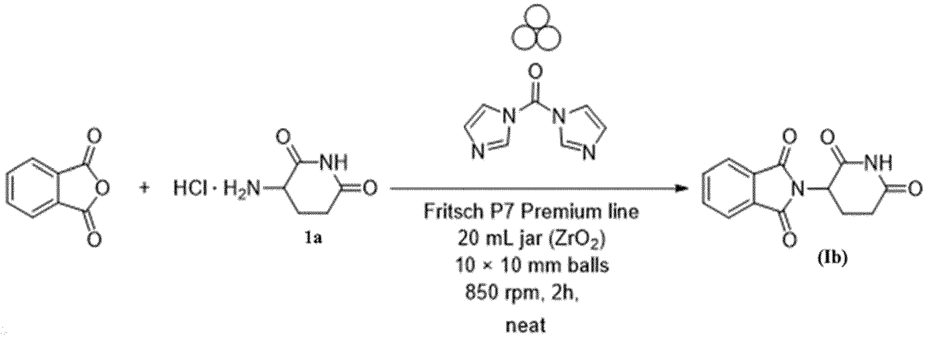

Phthalic anhydride (301 mg, 2.00 mmol, 1.1 equiv.), 3-aminopiperidine-2,6-dione hydrochloride (301 mg, 1.83 mmol, 1.0 equiv.) and 1,1'-carbonyldiimidazole (325 mg, 2.01 mmol, 1.1 equiv.) were loaded into a 20 mL zirconium oxide jar containing 10 × 10 mm ø zirconium oxide balls. The jar was closed and transferred into a Fritsch P7 Premium Line planetary mill, followed by milling at 850 rpm for 120 minutes to obtain a crude solid. After opening the jar, water (5 mL) was added to the crude solid to obtain a precipitate and said precipitate was filtered and further washed with water (3 × 10 mL) to obtain a white solid. Said white solid was dried in a vacuum oven overnight to obtain thalidomide (**1b**) (400 mg, 1.55 mmol, 85%).

**Thin layer chromatography** (AcOEt/*n*-heptane, 1:1 v/v) R_{f} = 0.26; **¹H NMR** (Figure 8, the number of hydrogen atoms are assigned as described in Figure 7) (500 MHz, DMSO-d₆) δ 11.14 (s, 1H, H-13), 7.98 - 7.87 (m, 4H, H-1 & H-2 & H-3 & H-6), 5.17 (dd, *J =* 12.8, 5.4 Hz, 1H, H-9), 2.96 - 2.84 (m, 1H, H-11a), 2.68 - 2.51 (m, 2H, H-10a & H-11b), 2.13 - 2.03 (m, 1H, H-10b). **¹³C NMR** (Figure 9, the number of carbon artoms are assigned as described in Figure 7) (126 MHz, DMSO-d₆) δ 173.2 (C-14), 170.3 (C-12), 167.6 (C-7 & C-8), 135.4 (C-1 & C-2), 131.7 (C-4 & C-5), 123.9 (C-3 & C-6), 49.5 (C-9), 31.4 (C-11), 22.5 (C-10). **ESI-MS** [(M + Na]⁺) calcd. for C₁₃H₁₀N₂NaO₄⁺: 281,05; found: 281,09 (Figure 10)

### Example 14 - Second example of preparation of thalidomide under planetary ball-milling (PBM)

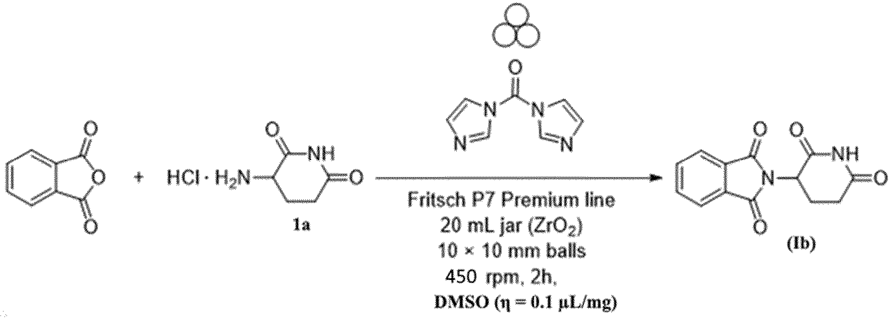

Phthalic anhydride (300 mg, 2.00 mmol, 1.1 equiv.), 3-aminopiperidine-2,6-dione hydrochloride (302 mg, 1.83 mmol, 1.0 equiv.) and 1,1'-carbonyldiimidazole (325 mg, 2.00 mmol, 1.1 equiv.) were loaded into a 20 mL zirconium oxide jar containing 10 × 10 mm ø zirconium oxide balls. Dimethyl sulfoxide (96 µL, η = 0.1 µL/mg) was added, after which the jar was closed and transferred into a Fritsch P7 Premium Line planetary mill. The jar was milled at 450 rpm for 60 minutes to obtain a crude solid. After opening the jar, water (5 mL) was added to the crude solid to form a precipitate, and said precipitate was filtered and further washed with water (3 × 10 mL) to obtain a white solid. Said white solid was dried in a vacuum oven overnight to obtain thalidomide (440 mg, 1.70 mmol, 93%). Thin-layer chromatography, ¹H NMR, ¹³C NMR and ESI-MS are the same data than those in Example 13.

### Example 15 - Third example of preparation of thalidomide under planetary ball-milling (PBM)

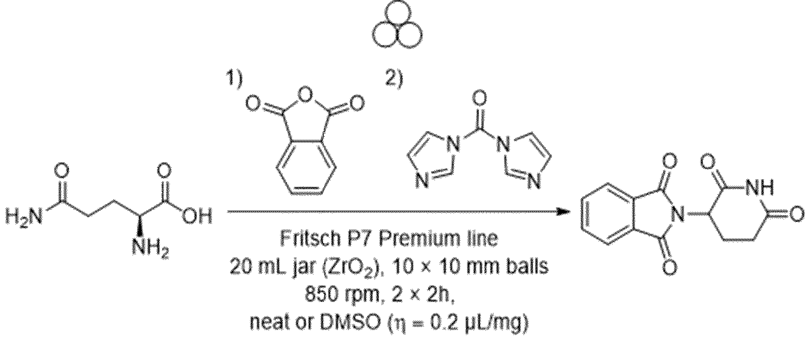

Phthalic anhydride (308 mg, 2.05 mmol, 1.0 equiv.) and L-glutamine (300 mg, 2.05 mmol, 1.0 equiv.) were loaded into a 20 mL zirconium oxide jar containing 10 × 10 mm ø zirconium oxide balls. The jar was closed and transferred into a Fritsch P7 Premium Line planetary mill, followed by milling at 850 rpm for 120 minutes. After opening the jar, 1,1'-carbonyldiimidazole (799 mg, 4.93 mmol, 2.4 equiv.) was added to the jar and the jar was closed. The jar was transferred into the planetary mill and milled at 850 rpm for 120 minutes. The jar was opened, and water (5 mL) was added to the crude solid to obtain a precipitate, and said precipitate was filtered and further washed with water (3 × 10 mL) to obtain a white solid. The resulting white solid was dried in a vacuum oven overnight to obtain thalidomide under the form of a white solid (420 mg, 1.63 mmol, 79%). Thin-layer chromatography, ¹H NMR, ¹³C NMR and ESI-MS are the same data than those in Example 13.

## Claims

1. Use of a mechanical force to generate a mechanochemical reaction in the implementation of a process of preparation in a reactor of a compound of formula (I): 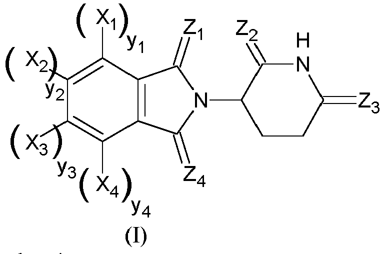 wherein:
- Z₁, Z₂, Z₃ and Z₄, identical or different, are chosen among: oxygen or sulfur atom, X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₅)acyl group, a 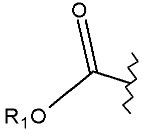 group, or a 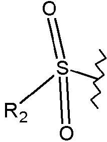 group
wherein R₁ is chosen among: hydrogen atom, (C₁-C₅)alkyl group, or -NR₁ₐR_{1b} group,
R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
or a pharmaceutically acceptable salt thereof, comprising n steps, n being 1 or 2, from 3-aminopiperidine-2,6-dione hydrochloride or L-glutamine, wherein at least one step is mechanochemical.

2. The use, according to claim 1, wherein the compound of formula (I) has a formula chosen among (Ia) or (Ib):

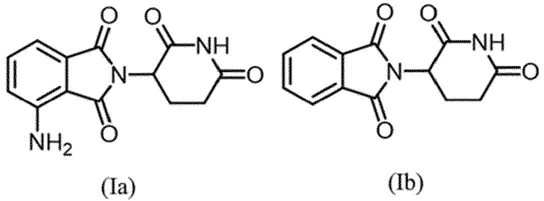

3. The use, according to any one of claims 1 or 2,
wherein said process is carried out in batch, in continuous or in semi-continuous, or
wherein the mechanical forces are generated by a method chosen among:
o milling, using a milling device, in particular vibrating ball-milling (VBM) or planetary ball-milling (PBM),
o resonant acoustic mixing (RAM),
o screw extrusion (single, twin or multi),
or
wherein one step or two steps are mechanochemical.

4. The use according to any one of claims 1 to 3, wherein said process of preparation can provide at least 4.10³ kg.m⁻³ of said reactor.day⁻¹, in particular from 4.10³ kg.m⁻³ of said reactor.day⁻¹ to 68 000 kg.m⁻³ of said reactor.day⁻¹, of said compound of formula (I) or of said pharmaceutically acceptable salt thereof.

5. A mechanochemical batch, continuous or semi-continuous process of preparation of a compound of formula (I), 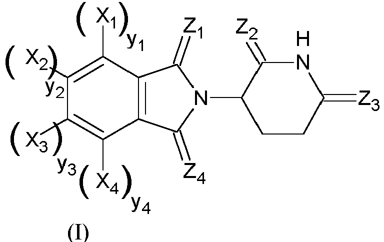 wherein:
- Z₁, Z₂, Z₃ and Z₄, identical or different, are chosen among: oxygen or sulfur atom, X₁, X₂, X₃ and X₄, identical or different, are chosen among: (C₁-C₅)alkyl group, (C₁-C₅)alkoxy group, amino group, halogen atom, trifluoromethyl group, hydroxy group, (H or C₁-C₅)acyl group, a 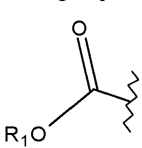 group, or a 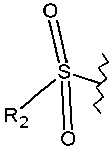 group
wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
a) a cycloimidation step using 3-aminopiperidine-2,6-dione hydrochloride or L-glutamine as starting material, in the presence of
a carboxylic function bearer chosen among 3-nitrophthalic acid, 3-nitrophthalic anhydride, phthalic acid or phthalic anhydride, and
optionally a coupling agent,
optionally a base, possibly generated *in situ* by said coupling agent, in particular chosen among tripotassium phosphate and potassium carbonate,
optionally at least one additive, in particular said at least one additive being under the form of a liquid or of a solid,
said cycloimidation step being carried out under neat grinding, or in the presence of a solid adjuvant, or in liquid-assisted grinding (LAG) conditions, or in LAG conditions with a solid adjuvant,
to obtain a crude product containing a compound of formula (II): 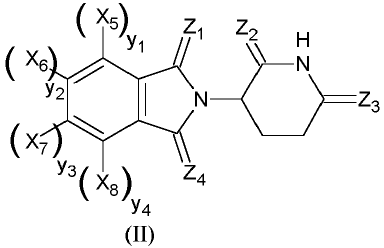 wherein:
- Z₁, Z₂, Z₃ and Z₄, identical or different, are chosen among : oxygen or sulfur atom, X₅, X₆, X₇ and X₈, identical or different, are chosen among: (C₁-C₅)alkyl group, (C₁-C₅)alkoxy group, nitro group, halogen atom, trifluoromethyl group, hydroxy group, (H or C₁-C₅)acyl group, a 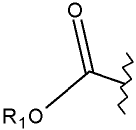 group, or a 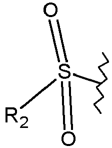 group
wherein R₁ is chosen among: hydrogen atom, (C₁-C₅)alkyl group, or -NR₁ₐR_{1b} group, R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising optionally,
b) a step of mechanochemical reduction of the above-mentioned compound of formula (II), with at least one additive, using mechanochemical forces, in liquid-assisted grinding (LAG) conditions with sodium dithionite, to obtain a crude product containing said compound of formula (I).

6. The batch, continuous or semi-continuous, mechanochemical process, according to claim 5, wherein the step a) is carried out with at least one additive, said additive being a solid additive or a liquid additive.
and
wherein the step a) is carried out with at least one additive, and:
• when said at least one additive is solid under mechanical forces, the step a) is carried out in the presence of a solid adjuvant,
or
• when said at least one additive is liquid under mechanical forces, the step a) is carried out in liquid-assisted grinding (LAG) conditions,
or
• when said at least one additive comprises a solid adjuvant and a liquid additive under mechanical forces, the step a) is carried out in LAG conditions with a solid adjuvant.

7. The mechanochemical batch, continuous or semi-continuous process, according to any one of claims 5 or 6, wherein in step a) and/or in step b), the mechanical forces are generated by a method chosen among:
o milling, using a milling device, in particular vibrating ball-milling (VBM) or planetary ball-milling (PBM),
o resonant acoustic mixing (RAM),
o screw extrusion (single, twin or multi).

8. The batch, continuous or semi-continuous, mechanochemical process, according to any one of claims 5 to 7, of preparation of a compound of formula (III): 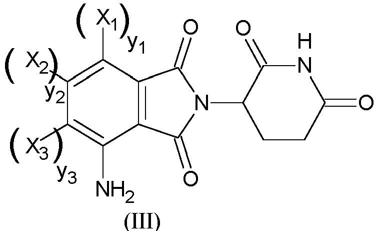 in particular of formula 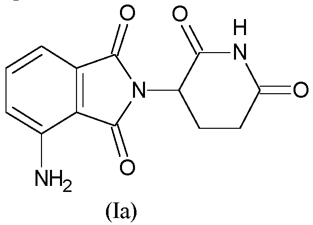
wherein X₁, X₂, X₃, y₁, y₂ and y₃ have the meaning defined above,
said process comprising:
a) a cycloimidation step, wherein 3-aminopiperidine-2,6-dione hydrochloride is used as starting material, in the presence of
a carboxylic function bearer chosen among 3-nitrophthalic acid or 3-nitrophthalic anhydride,
a coupling agent chosen among carbonyldiimidazole (CDI), *N,N,N,N-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-ethyl-3-(3-dimethylaminopropyl)carbodiimide (EDC),
and optionally a base, possibly generated *in situ* by said coupling agent,
optionally at least one additive is used, in particular dimethyl sulfoxide or dimethyl isosorbide, or a mixture of thereof,
and said mechanical forces are generated by a milling device, and said cycloimidation step is carried out under neat grinding or in LAG conditions,
to obtain a crude product containing a compound of formula (IV), 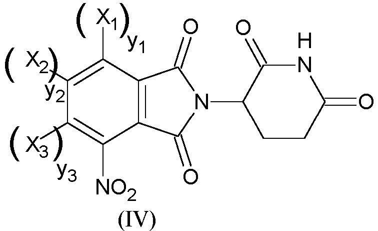
wherein X₁, X₂, X₃, y₁, y₂ and y₃ have the meaning defined above,
wherein when the step a) is in LAG conditions, the latter are defined by the parameter η that is greater than 0 µL/mg and less than or equal to 1 µL/mg, more preferably is comprised from about 0.1 µL/mg to 0.3 µL/mg, more preferably 0.2 µL/mg,
said process comprising
b) a step of mechanochemical reduction of the above-mentioned compound of formula (IV), said mechanical forces being generated by a milling device, to obtain a crude product containing said compound of formula (III),
wherein in the step b), the LAG conditions are defined by the parameter η that is greater than 0 µL/mg and less than or equal to 1 µL/mg, more preferably is comprised from about 0.25 µL/mg to 0.75 µL/mg, more preferably 0.5 µL/mg.

9. The batch mechanochemical process according to claim 8, wherein:
in step a), the milling device is chosen among VBM or PBM,
and/or
in step b), the milling device is chosen among VBM or PBM.

10. The batch, continuous or semi-continuous, mechanochemical process, according to any one of claims 5 to 7, of preparation of a compound of formula (III): 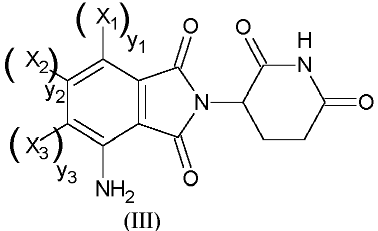 in particular of formula 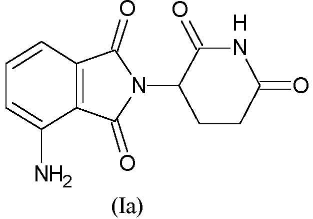
wherein X₁, X₂, X₃, y₁, y₂ and y₃ have the meaning defined above,
said process comprising:
a) a cycloimidation step, wherein 3-aminopiperidine-2,6-dione hydrochloride is used as starting material, in the presence of
a carboxylic function bearer chosen among 3-nitrophthalic acid or 3-nitrophthalic anhydride,
a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-Ethyl-3-(3-dimethylaminopropyl)carbodiimide (EDC)
and optionally a base, possibly generated *in situ* by said coupling agent,
optionally at least one liquid additive is used, in particular dimethyl sulfoxide or dimethyl isosorbide, or a mixture of thereof,
and said mechanical forces are generated by RAM and said cycloimidation step is carried out in LAG conditions, to obtain a crude product containing a compound of formula (IV), 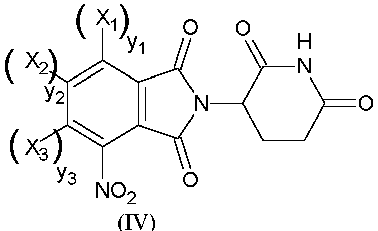 wherein X₁, X₂, X₃, y₁, y₂ and y₃ have the meaning defined above,
wherein in the step a), the LAG conditions are defined by the parameter η that is greater than 0 µL/mg and less than or equal to 1 µL/mg, more preferably is comprised from about 0.2 µL/mg to 0.8 µL/mg, more preferably 0.5 µL/mg,
said process comprising
b) a step of mechanochemical reduction of the above-mentioned compound of formula (IV), said mechanical forces being generated by RAM, to obtain a crude product containing said compound of formula (III),
wherein in the step b), the LAG conditions are defined by the parameter η that is greater than 0 µL/mg and less than or equal to 1 µL/mg, more preferably is comprised from about 0.60 µL/mg to 0.90 µL/mg, more preferably 0.75 µL/mg.

11. The batch, continuous or semi-continuous, mechanochemical process according to claim 10, wherein:
in step a), the g force is comprised from 2 g to 100 g, in particular 90 g.
and/or
in step b), the g force is comprised from 2 g to 100 g, in particular 90 g.

12. The continuous mechanochemical process, according to any one of claims 5 to 7, of preparation of preparation of a compound of formula (III): 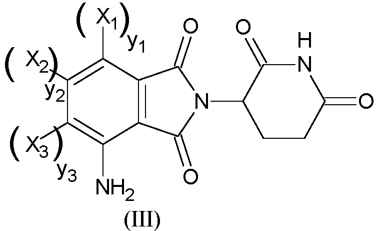 in particular of formula 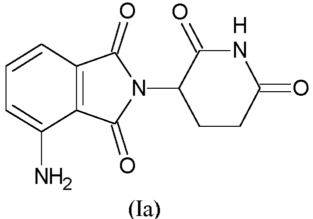
wherein X₁, X₂, X₃, y₁, y₂ and y₃ have the meaning defined above,
said process comprising:
a) a cycloimidation step, wherein 3-aminopiperidine-2,6-dione hydrochloride is used as starting material, in the presence of
a carboxylic function bearer which is 3-nitrophthalic acid or 3-nitrophthalic anhydride, and optionally at least one additive is used, in particular dimethyl sulfoxide or dimethyl isosorbide, or a mixture of thereof,
and optionally a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), 1-Ethyl-3-(3-dimethylaminopropyl)carbodiimide (EDC),
and optionally a base, possibly generated *in situ* by said coupling agent, and,
said cycloimidation step is carried out under neat grinding, to obtain a crude product containing a compound of formula (IV), 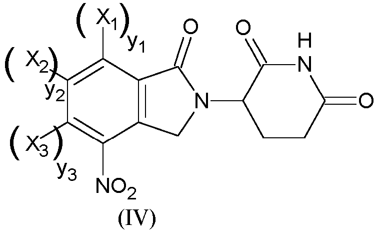 wherein X₁, X₂, X₃, y₁, y₂ and y₃ have the meaning defined above,
in particular the mechanical forces are generated by twin screw extrusion,
said process comprising
b) a step of mechanochemical reduction of the above-mentioned compound of formula (IV), to obtain a crude product containing said compound of formula (III), in particular the mechanical forces are generated by twin screw extrusion,
wherein in the step b), the LAG conditions are defined by the parameter η that is greater than 0 mL/g and less than or equal to 1 mL/g, more preferably is comprised from about 0.50 mL/g to 1 mL/g, more preferably 0.75 mL/g,

13. The batch, continuous or semi-continuous, mechanochemical process, according to any one of claims 5 to 7, of preparation of a compound of formula (VII): 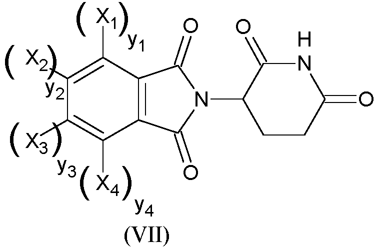 in particular of formula 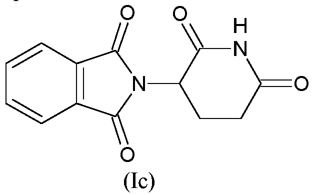 wherein:
X₁, X₂, X₃ and X₄, identical or different, are chosen among: (C₁-C₅)alkyl group, (C₁-C₅)alkoxy group, halogen atom, trifluoromethyl group, hydroxy group, (H or C₁-C₅)acyl group, a 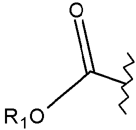 group, or a 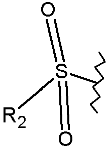 group
wherein R₁ is chosen among: hydrogen atom, (C₁-C₅)alkyl group, or -NR₁ₐR_{1b} group,
R₁ₐ and R_{1b}, identical or different, are chosen among: hydrogen atom or (C₁-C₅)alkyl group,
and R₂ is chosen among: hydroxy group, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
b) a cycloimidation step wherein 3-aminopiperidine-2,6-dione hydrochloride is used as starting material, in the presence of
a carboxylic function bearer chosen among phthalic acid or phthalic anhydride,
a coupling agent chosen among carbonyldiimidazole (CDI), *N,N,N,N-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), in particular carbonyldiimidazole (CDI), 1-Ethyl-3-(3-dimethylaminopropyl)carbodiimide (EDC), and
a base, possibly generated *in situ* by said coupling agent, and
optionally at least one additive is used, in particular dimethyl sulfoxide,
and said mechanical forces are generated by a milling device and said cycloimidation step is carried out under neat grinding, or with or without an adjuvant, or in LAG conditions with or without an adjuvant,
to obtain a crude product containing said compound of formula (VIII),
wherein when the step a) is in LAG conditions, the latter are defined by the parameter η that is greater than 0 µL/mg and less than or equal to 1 µL/mg, more preferably is comprised from about 0.05 µL/mg to 0.35 µL/mg, more preferably 0.1 µL/mg.

14. The batch, continuous or semi-continuous, mechanochemical process, according to any one of claims 5 to 7, of preparation of a compound of formula (VII): 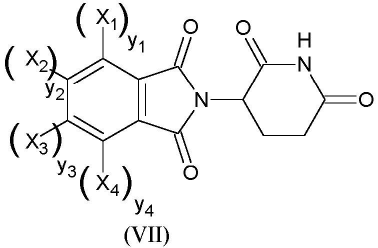 in particular of formula 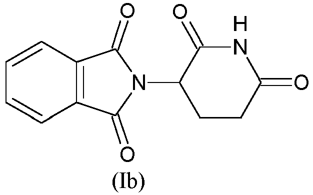 wherein :
X₁, X₂, X₃ and X₄, identical or different, are chosen among: (C₁-C₅)alkyl group, (C₁-C₅)alkoxy group, halogen atom, trifluoromethyl group, hydroxy group, (H or C₁-C₅)acyl group, a 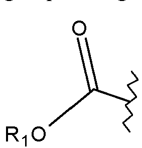 group, or a 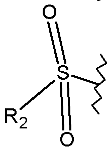 group
wherein R₁ is chosen among: hydrogen atom, (C₁-C₅)alkyl group, or -NR₁ₐR_{1b} group,
R₁ₐ and R_{1b}, identical or different, are chosen among: hydrogen atom or (C₁-C₅)alkyl group,
and R₂ is chosen among: hydrogen atom, (C₁-C₅)alkoxy group, -NR₂ₐR_{2b},
R₂ₐ and R_{2b}, identical or different, are chosen among: hydrogen atom or (C₁-C₅)alkyl group
- y₁, y₂, y₃ and y₄, identical or different, are equal to 0 or 1, and provided that y₁ + y₂ + y₃ + y₄ is strictly inferior to 3,
said process comprising:
a) a cycloimidation step, wherein L-glutamine is used as starting material, in the presence of
a carboxylic function bearer which is phthalic anhydride,
a coupling agent chosen among carbonyldiimidazole (CDI), *N,N,N',N'-*tetramethylchloroformamidinium hexafluorophosphate (TCFH), 1-[bis(dimethylamino)methylene]-1H-1,2,3-triazolo[4,5-b]pyridinium 3-oxide hexafluorophosphate (HATU), (1-cyano-2-ethoxy-2-oxoethylidenaminooxy)dimethylamino-morpholino-carbenium hexafluorophosphate (COMU), dicyclohexylcarbodiimide (DCC), in particular carbonyldiimidazole (CDI), 1-Ethyl-3-(3-dimethylaminopropyl)carbodiimide (EDC), and
a base, possibly generated *in situ* by said coupling agent, and
optionally at least one additive is used, in particular dimethyl sulfoxide,
and said mechanical forces are generated by a milling device and said cycloimidation step is carried out under neat grinding, or with or without an adjuvant, or in LAG conditions with or without an adjuvant, to obtain a crude product containing said compound of formula (VII),
wherein when the step a) is in LAG conditions, the latter are defined by the parameter η that is greater than 0 µL/mg and less than or equal to 1 µL/mg, more preferably is comprised from about 0.05 µL/mg to 0.35 µL/mg, more preferably 0.2 µL/mg.

15. The batch, continuous or semi-continuous, mechanochemical process according to any one of claim 13 or 14, wherein the step a) is carried out in the presence of CDI as coupling agent, and imidazole as a base, generated *in situ* from CDI.
